(19) Europäisches Patentamt
European Patent Office
Office européen des brevets

(11) Veröffentlichungsnummer: **0 210 471 B1**

# (12) EUROPÄISCHE PATENTSCHRIFT

(45) Veröffentlichungstag der Patentschrift: **07.10.92**

(21) Anmeldenummer: **86109185.8**

(22) Anmeldetag: **04.07.86**

(51) Int. Cl.5: **C08G 12/02**, C08G 12/10, C08G 12/40, C07D 239/10, C09D 161/34, C09D 5/44

## (54) Umsetzungsprodukte und Kondensationsprodukte auf Basis substituierter Propylenharnstoffe, deren Herstellung und Verwendung.

(30) Priorität: **17.07.85 DE 3525438**
**17.07.85 DE 3525434**
**17.07.85 DE 3525437**
**17.07.85 DE 3525435**

(43) Veröffentlichungstag der Anmeldung:
**04.02.87 Patentblatt 87/06**

(45) Bekanntmachung des Hinweises auf die Patenterteilung:
**07.10.92 Patentblatt 92/41**

(84) Benannte Vertragsstaaten:
**AT DE FR GB IT NL SE**

(56) Entgegenhaltungen:
**GB-A- 1 069 526**
**US-A- 3 772 225**
**US-A- 4 284 758**
**US-A- 4 349 663**

(73) Patentinhaber: **BASF Aktiengesellschaft**
**Carl-Bosch-Strasse 38**
**W-6700 Ludwigshafen(DE)**

(72) Erfinder: **Goeckel, Ulrich, Dr.**
**Leipziger Strasse 6**
**W-6737 Boehl-Iggelheim(DE)**
Erfinder: **Petersen, Harro, Dr.**
**Kalmitstrasse 23**
**W-6710 Frankenthal(DE)**
Erfinder: **Osterloh, Rolf, Dr.**
**Am Wehrhaus 16a**
**W-6718 Gruenstadt(DE)**
Erfinder: **Schupp, Eberhard, Dr.**
**Tilsiter Weg 4**
**W-6830 Schwetzingen(DE)**
Erfinder: **Loch, Werner, Dr.**
**In der Bleiche 2**
**W-6701 Erpolzheim(DE)**
Erfinder: **Schwerzel, Thomas, Dr.**
**Budapester Strasse 51**
**W-6700 Ludwigshafen(DE)**

**EP 0 210 471 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Kondensationsprodukte auf Basis substituierter Propylenharnstoffe, die mindestens zwei cyclische Harnstoffeinheiten enthalten, Umsetzungsprodukte dieser Kondensationsprodukte mit Di- und/oder Polyisocyanaten, Derivaten von Di- und/oder Polycarbonsäuren oder p-substituierten Phenolen und/oder deren O-Methylolderivate, Verfahren zu ihrer Herstellung und ihre Verwendung als Komponente in hitzehärtbaren Überzugsmitteln.

Monocyclische Propylenharnstoffe sind in der Literatur beschrieben, so z.B. in Angew. Chem. 76, 909 (1964), DE-PS 12 30 805, Monatsheft Chem. 96, 1950 (1965) oder DE-PS 12 31 24 bzw. DE-PS 12 29 093.

N-Methylolderivate bzw. -alkoximethylverbindungen monocyclischer Propylenharnstoffe mit 4-ständiger Hydroxi- oder Alkoxifunktion werden technisch als Vernetzer zur Knitterfreiausrüstung cellulosehaltiger Textilien oder als Komponenten in Lackbindemitteln eingesetzt. Produkte dieses Typs benötigen jedoch zur Vernetzung bei vergleichsweise niedrigen Temperaturen saure Katalysatoren. Nachteilig ist vor allem in technischen Produkten die Anwesenheit von freiem Formaldehyd und die Freisetzung von Formaldehyd während der Applikation und aus den vernetzten Substraten. Gleiches gilt für den Einsatz von Aminoplast-Formaldehyd-Harzen, wie z.B. veretherten Harnstoff- oder Melamin-Formaldehyd-Harzen als Vernetzer für Lackbindemittel. Zur Aushärtung benötigen diese Systeme wiederum Säurekatalyse oder aber vergleichsweise hohe Temperaturen. Für Anwendungszwecke, die eine Vernetzung ohne Säurekatalyse erfordern, wie beispielsweise die kathodische Elektrotauchlackierung, sind diese Harze daher nur in Ausnahmefällen und bei hoher Härtungstemperatur oberhalb 180 °C brauchbar. Das in der DE-AS 20 57 799 beschriebene Verfahren zur kathodischen elektrophoretischen Abscheidung von in Wasser dispergierten, ionischen, organischen Harzen beinhaltet die Verwendung blockierter, multifunktioneller Isocyanate als Vernetzungskomponente. Die hierin genannten, in der Praxis verwendbaren Blockierungsmittel, wie aliphatische Alkohole und auch z.B. Caprolactam bedingen relativ hohe Härtungstemperaturen von über 180 °C, den Einsatz von beispielsweise Zinnsalzen als Vernetzungskatalysatoren und einen beträchtlichen Verlust flüchtiger, organischer Stoffe aus dem Lackfilm während des Härtungsvorgangs.

Aufgabe der vorliegenden Erfindung war es daher, diese Nachteile zu beheben und für verschiedene Anwendungsgebiete Harze zur Verfügung zu stellen, die ohne die Anwesenheit saurer Katalysatoren oder von Schwermetallsalzen bei vergleichsweise niedrigen Temperaturen makromolekulare Bindemittel mit geeigneten funktionellen Gruppen, wie beispielsweise Hydroxyl-oder Aminogruppen, wirksam vernetzen, ohne toxische und umweltbelastende Stoffe, wie vor allem Formaldehyd, Amine, Phenol oder ähnliche freizusetzen. Diese Aufgabe konnte gelöst werden durch harzartige Kondensationsprodukte auf Basis von Propylenharnstoffen mit mindestens 2 substituierten Propylenharnstoffeinheiten mit reaktiver 4-ständiger Gruppierung.

Gegenstand der vorliegenden Erfindung sind Kondensationsprodukte die im wesentlichen substituierte Propylenharnstoffe der allgemeinen Formeln (I) und/oder (II)

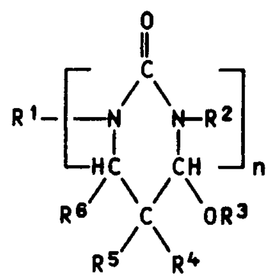

(I)

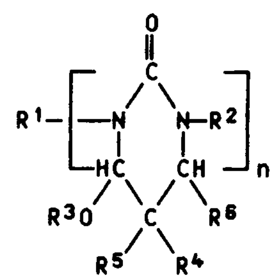

(II)

enthalten, worin $R^1$ einen mindestens zweiwertigen organischen, 4 bis 60 Kohlenstoffatome enthaltenden geradkettigen oder verzweigten Alkylen-, Cycloalkylen-, Oxaalkylen- oder Azaalkylenrest bedeutet, wobei der Rest $R^1$ gegebenenfalls eine oder mehrere Hydroxylgruppen und/oder mit Hydroxyalkyl-, Hydroxycycloalkyl-, Hydroxyoxaalkyl- und/oder Hydroxyazaalkylgruppen substituierte Harnstoff-, Carbamat-, Carbonamid- oder Sulfonamidgruppen enthalten kann,

$R^2$, $R^3$, $R^4$, $R^5$ und $R^6$ untereinander gleich oder verschieden sind und für Wasserstoff, Alkylreste mit 1 bis 18 Kohlenstoffatomen, Cycloalkylreste mit 5 bis 15 Kohlenstoffatomen, Arylreste mit 6 bis 15 Kohlenstoffatomen, Aralkylreste mit 7 bis 16 Kohlenstoffatomen, Oxaalkyl- oder Azaalkylreste mit 2 bis 18 Kohlenstoffatomen, stehen,

und $R^2$ zusätzlich für einen Hydroxyalkylrest stehen kann, mit der Maßgabe, daß für den Fall, daß $R^2$ für einen Hydroxyalkylrest steht, dieser 4 bis 18 Kohlenstoffatome enthält, und $R^2$ nicht für einen Aryl-oder Aralkylrest steht,
und n = 2 bis 20 ist, wobei die Wertigkeit von $R^1$ n entspricht und die einzelnen Reste $R^2$ bis $R^6$ der n Propylenharnstoffeinheiten der Formeln (I) und/oder (II) innerhalb der angegebenen Definitionen untereinander jeweils gleich oder verschieden sein können.

Gegenstand der Erfindung ist auch ein Verfahren zur Herstellung dieser Kondensationsprodukte, das dadurch gekennzeichnet ist, daß man zunächst

a) primäre Di- und/oder Polyamine der allgemeinen Formel (III)

$$R^1-[NH_2]_n$$

mit

b) Harnstoff, monosubstiutierten oder symmetrisch disubstituierten Harnstoffen der allgemeinen Formel (IV) $R^2NH-CO-NHR^2$

bei Temperaturen von 100 bis 200 °C, gegebenenfalls in Anwesenheit von sauren oder basischen Katalysatoren sowie gegebenenfalls in Anwesenheit von

c) Dihydroxiverbindungen, Polyhydroxiverbindungen, Alkanolaminen, Hydroxyalkylcarbonsäuren und/oder Hydroxyalkylsulfonsäuren oder Hydroxyalkylsulfonsäureestern

umsetzt und die so erhaltenen Produkte mit

d) 1,4 bis 2,5 Mol eines oder mehrerer Aldehyde der allgemeinen Formel (V)

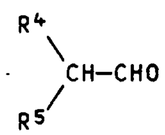

und/oder (VI) $R^6$—CHO

pro Mol Harnstoff (b) in Gegenwart saurer Katalysatoren bei Temperaturen von 50 bis 150 °C, gegebenenfalls in Anwesenheit eines Lösungsmittels umsetzt, mit der Maßgabe, daß mindestens 0,7 Mol pro Mol eingesetztem Harnstoff (b) eines Aldehyds (d) verwendet werden, der mindestens ein in $\alpha$-Stellung zur Aldehydgruppe befindliches Wasserstoffatom enthält,

wobei $R^1$ bis $R^6$ und n die oben angegebene Bedeutung haben;
sowie ein Verfahren zur Herstellung dieser Kondensationsprodukte, das dadurch gekennzeichnet ist, daß man

a) primäre Monoamine der allgemeinen Formel (VII) $R^2-NH_2$ oder primäre Di- und/oder Polyamine der allgemeinen Formel (III)

$$R^1-[NH_2]_n \quad ,$$

zunächst mit

e) Monoisocyanaten der allgemeinen Formel (VIII) $R^2-NCO$ oder Di- und/oder Polyisocyanaten der allgemeinen Formel (IX)

$$R^1-[NCO]_n$$

umsetzt mit der Maßgabe, daß für den Fall, daß als Komponente (a) primäre Monoamine $R^2-NH_2$ eingesetzt werden, diese mit Di- und/oder Polyisocyanaten

$$R^1-[NCO]_n$$

umgesetzt werden, für den Fall, daß als Komponente (e) Monoisocyanate $R^2$-NCO eingesetzt werden, diese mit Di- und/oder Polyaminen

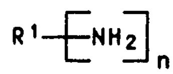

umgesetzt werden und die so erhaltenen Di- oder Polyharnstoffe mit

d) 1,4 bis 2,5 Mol eines oder mehrerer Aldehyde

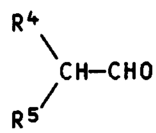 und/oder 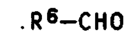

pro Mol der bei der Umsetzung von (a) mit (e) gebildeten Harnstoffeinheiten in Gegenwart saurer Katalysatoren bei Temperaturen von 50 bis 150 °C, gegebenenfalls in Anwesenheit eines Lösungsmittels, umsetzt, mit der Maßgabe, daß mindestens 0,7 Mol pro Mol der durch Umsetzung von a) mit e) gebildeten Harnstoffeinheiten mindestens eines Aldehyds d) verwendet werden, der mindestens ein in $\alpha$-Stellung zur Aldehydgruppe befindliches Wasserstoffatom enthält,

wobei $R^1$ bis $R^6$ und n die oben angegebene Bedeutung haben.

Vorzugsweise wird als Aldehyd d) Isobutyraldehyd oder ein Gemisch von Isobutyraldehyd mit Formaldehyd verwendet.

Gegenstand der vorliegenden Erfindung sind insbesondere auch Umsetzungsprodukte von Kondensationsprodukten, die im wesentlichen substituierte Propylenharnstoffe der allgemeinen Formeln (I) und/oder (II)

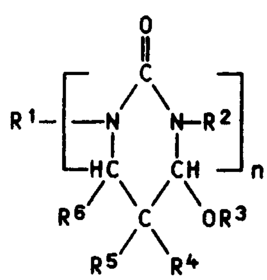

(I)

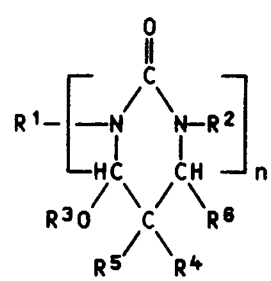

(II)

enthalten, worin $R^1$ einen mindestens zweiwertigen organischen, 4 bis 60 Kohlenstoffatome enthaltenden geradkettigen oder verzweigten Alkylen-, Cycloalkylen-, Oxaalkylen- oder Azaalkylenrest bedeutet, wobei der Rest $R^1$ gegebenenfalls eine oder mehrere Hydroxylgruppen und/oder mit Hydroxyalkyl-, Hydroxycycloalkyl-, Hydroxyoxaalkyl- und/oder Hydroxyazaalkylgruppen substituierte Harnstoff-, Carbamat-, Carbonamid- oder Sulfonamidgruppen enthalten kann,

$R^2$, $R^3$, $R^4$, $R^5$ und $R^6$ untereinander gleich oder verschieden sind und für Wasserstoff, Alkylreste mit 1 bis 18 Kohlenstoffatomen, Cycloalkylreste mit 5 bis 15 Kohlenstoffatomen, Arylreste mit 6 bis 15 Kohlenstoffatomen, Aralkylreste mit 7 bis 16 Kohlenstoffatomen, Oxaalkyl- oder Azaalkylreste mit 2 bis 18 Kohlenstoffatomen, stehen,

und $R^2$ zusätzlich für einen Hydroxyalkylrest stehen kann, mit der Maßgabe, daß für den Fall, daß $R^2$ für einen Hydroxyalkylrest steht, dieser 4 bis 18 Kohlenstoffatome enthält, und $R^2$ nicht für einen Aryl- oder Aralkylrest steht,

und n = 2 bis 20 ist, wobei die Wertigkeit von $R^1$ n entspricht und die einzelnen Reste $R^2$ bis $R^6$ der n Propylenharnstoffeinheiten der Formeln (I) und/oder (II) innerhalb der oben angegebenen Definitionen untereinander jeweils gleich oder verschieden sein können, mit

Di- und/oder Polyisocyanaten,

Derivaten von Di- und/oder Polycarbonsäuren oder

p-substituierten Phenolen und/oder deren o-Methylolderivaten, mit der Maßgabe, daß bei Verwendung von

o-Methylolphenolen mindestens einer der Reste $R^1$ oder $R^2$ der Formeln (I) und/oder (II) eine Hydroxylgruppe enthält oder der Rest $R^2$ für Wasserstoff steht.

Desgleichen sind Verfahren zur Herstellung dieser Umsetzungsprodukte Gegenstand der Erfindung, nämlich ein Verfahren zur Herstellung dieser Umsetzungssprodukte wobei man zunächst

a) primäre Di- und/oder Polyamine der allgemeinen Formel (III)

$$R^1\text{—}[NH_2]_n$$

mit

b) Harnstoff, monosubstituierten oder symmetrisch disubstituierten Harnstoffen der allgemeinen Formel (IV) $R^2NH\text{-}CO\text{-}NHR^2$

bei Temperaturen von 100 bis 200 °C, gegebenenfalls in Anwesenheit von sauren oder basischen Katalysatoren sowie gegebenenfalls in Anwesenheit von

c) Dihydroxiverbindungen, Polyhydroxiverbindungen, Alkanolaminen, Hydroxyalkylcarbonsäuren und/oder Hydroxyalkylsulfonsäuren oder Hydroxyalkylsulfonsäureestern

umsetzt und die so erhaltenen Di- und/oder Polyharnstoffe mit

d) 1,4 bis 2,5 Mol eines oder mehrerer Aldehyde der allgemeinen Formel (V)

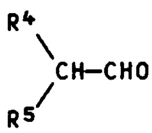 und/oder (VI) $R^6$—CHO

pro Mol Harnstoff (b) in Gegenwart saurer Katalysatoren bei Temperaturen von 50 bis 150 °C, gegebenenfalls in Anwesenheit eines Lösungsmittels umsetzt, mit der Maßgabe, daß mindestens 0,7 Mol pro Mol eingesetztem Harnstoff (b) eines Aldehyds (d) verwendet werden, der mindestens ein in $\alpha$-Stellung zur Aldehydgruppe befindliches Wasserstoffatom enthält,

wobei $R^1$ bis $R^6$ und n die oben angegebene Bedeutung haben,

und die so erhaltenen Produkte mit

Di- und/oder Polyisocyanaten,

Derivaten von Di- und/oder Polycarbonsäuren oder

p-substituierten Phenolen und/oder deren o-Methylolderivaten umsetzt, mit der Maßgabe, daß pro Mol eingesetzten Harnstoffs b) 0,25 bis 1,0 Mol Isocyanatgruppen des Di- und/oder Polyisocyanats, Säurehalogenid- und/oder -anhydridgruppen oder p-substituiertes Phenol und/oder dessen o-Methylolderivat verwendet werden;

sowie ein Verfahren zur Herstellung dieser Umsetzungsprodukte, wobei man

a) primäre Monoamine der allgemeinen Formel (VII) $R^2\text{-}NH_2$ oder primäre Di- und/oder Polyamine der allgemeinen Formel (III)

$$R^1\text{—}[NH_2]_n$$

zunächst mit

e) Monoisocyanaten der allgemeinen Formel (VIII) $R^2\text{-}NCO$ oder Di-und/oder Polyisocyanaten der allgemeinen Formel (IX)

$$R^1\text{—}[NCO]_n$$

umsetzt mit der Maßgabe, daß für den Fall, daß als Komponente (a) primäre Monoamine $R^2\text{-}NH_2$ eingesetzt werden, diese mit Di- und/oder Polyisocyanaten

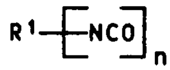

umgesetzt werden, und für den Fall, daß als Komponente (e) Monoisocyanate $R^2$-NCO eingesetzt werden, diese mit Di- und/oder Polyaminen

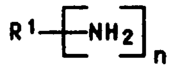

umgesetzt werden und die so erhaltenen Di- oder Polyharnstoffe mit
d) 1,4 bis 2,5 Mol eines oder mehrerer Aldehyde

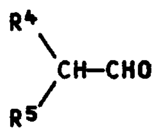 und/oder $R^6-CHO$

pro Mol der bei der Umsetzung von (a) mit (e) gebildeten Harnstoffeinheiten in Gegenwart saurer Katalysatoren bei Temperaturen von 50 bis 150 °C, gegebenenfalls in Anwesenheit eines Lösungsmittels, umsetzt, mit der Maßgabe, daß mindestens 0,7 Mol pro Mol der durch Umsetzung von a) mit e) gebildeten Harnstoffeinheiten mindestens eines Aldehyds d) verwendet werden, der mindestens ein in $\alpha$-Stellung zur Aldehydgruppe befindliches Wasserstoffatom enthält, wobei $R^1$ bis $R^6$ und n die oben angegebene Bedeutung haben, und die so erhaltenen Produkte mit
Di- und/oder Polyisocyanaten,
Derivaten von Di- und/oder Polycarbonsäuren oder
p-substituierten Phenolen und/oder deren o-Methylolderivaten umsetzt, mit der Maßgabe, daß pro Mol eingesetzten Harnstoffs b) 0,25 bis 1,0 Mol Isocyanatgruppen des Di- und/oder Polyisocyanats, Säurehalogenid und oder -anhydridgruppen oder p-substituiertes Phenol und/oder dessen o-Methylolderivat verwendet werden.

Vorzugsweise wird dabei als Aldehyd d) Isobutyraldehyd oder ein Gemisch von Isobutyraldehyd mit Formaldehyd verwendet.

Gegenstand der vorliegenden Erfindung sind außerdem hitzehärtbare Überzugsmittel, die als Bindemittel ein Gemisch aus
(A) mindestens einem Polyadditions-, Polykondensations- oder Polymerisationsprodukt mit einem mittleren Molekulargewicht $\overline{M}_n$ von 500 bis 10 000, welches im Mittel pro Molekül mindestens zwei Hydroxylgruppen sowie zusätzlich gegebenenfalls primäre und/oder sekundäre und/oder tertiäre Aminogruppen besitzt und
(B) einem Umsetzungsprodukt nach Anspruch 1 oder einem Kondensationsprodukt nach Anspruch 2
enthalten.

Als Komponente (A) bevorzugt sind Polyesterharze, Umsetzungsprodukte eines Epoxidharzes mit mindestens einem Amin, Alkohol oder Mercaptan sowie hydroxylgruppenhaltige Polyacrylatharze.

Gegenstand der Erfindung sind auch die Verwendung dieser Überzugsmittel für lösungsmittelhaltige Einbrennlacke, die daraus nach zumindest teilweiser Neutralisation mit einer Säure erhältlichen wasserverdünnbaren Produkte, deren Verwendung zur Herstellung kathodisch abscheidbarer Elektrotauchlacke und für wäßrige Einbrennlacke, sowie ein wäßriges Lackbad für die kathodische Elektrotauchlackierung, das 5 bis 30 Gew.% eines derartigen erfindungsgemäßen Überzugsmittels enthält.

Die Vernetzung erfolgt bevorzugt mit OH-gruppenhaltigen makromolekularen Bindemitteln unter Bildung der entsprechenden acetalischen Strukturen, wobei lediglich Wasser oder der dem Rest $R^3$ entsprechende Alkohol während der Härtung aus dem Vernetzer freigesetzt wird.

Überraschenderweise wurde gefunden, daß die Vernetzungsreaktion auch ohne Säurekatalyse bei sehr niedrigen Temperaturen von bereits ab 100 °C (während 20 Minuten) abläuft und beispielsweise mit üblichen Lackbindemitteln lösungsmittelbeständige und mechanisch feste Überzüge oder Lackfilme ergibt. Die Vernetzung kann dabei sowohl in saurem, als auch neutralen oder alkalischem Medium erfolgen, so daß nicht nur die Applikation aus wäßrigem oder lösungsmittelhatigen Systemen, sondern auch durch kathodische Elektrotauchlackierung möglich ist. Trotz der hohen Reaktivität gegenüber hydroxylgruppenhaltigen

Elektrotauchlackbindemitteln besitzt die Kombination aus Bindemittel und den erfindungsgemäßen Harzen eine überraschend hohe Lagerstabilität in wäßriger oder alkoholischer Lösung von mehreren Monaten. Die erfindungsgemäßen Kondensationsprodukte sind harzartige Substanzen mit Molekulargewichten, die im Mittel 300 bis 3000 betragen, und enthalten 2 bis 20, vorzugsweise 2 bis 10 substituierte Propylenharnstoffeinheiten. Die Produkte können in ihren Eigenschaften, wie Löslichkeit oder Verträglichkeit, Viskosität, Flexibilität und dergleichen, in weiten Bereichen über die verwendeten Substituenten beeinflußt werden.

In den allgemeinen Formeln (I) und/oder (II) kann $R^1$ einen mindestens zweiwertigen organischen, 4 bis 60, vorzugsweise 4 bis 15 Kohlenstoffatome enthaltenden geradkettigen oder verzweigten Alkylen-, Cycloalkylen-, Oxaalkylen- oder Azaalkylenrest bedeuten, wobei der Rest $R^1$ gegebenenfalls eine oder mehrere Hydroxylgruppen und/oder mit Hydroxyalkyl-, Hydroxycycloalkyl-, Hydroxyoxaalkyl- und/oder Hydroxyazaalkylgruppen substituierte Harnstoff-, Carbamat-, Carbonamid- oder Sulfonamidgruppen enthalten kann, und die Wertigkeit des Restes $R^1$ n entspricht.

So kann als Rest $R^1$ grundsätzlich jede organische Gruppierung dienen, die beispielsweise einem primären Di- und/oder Polyamin oder einem mehrfunktionellen Isocyanat zugrundeliegt, wie z.B. 1,4-Tetramethylen-, 1,5-Pentamethylen-, 1,6-Hexamethylen-, 1,8-Octamethylen-, 1,10-Decamethylen-und analoge Reste. Die Reste $R^1$ können Alkylverzweigungen aufweisen, wie beispielsweise der 2-Methyl-1,5-pentamethylen- oder der 2,2,5-Trimethyl-1,6-hexamethylenrest. Geeignet sind auch Cycloalkylengruppen, vorzugsweise solche mit 5 bis 8, insbesondere 6 Ringgliedern, wie etwa der Cyclohexylenrest, 4,4'-Dicyclohexyl-($C_1$- bis $C_8$-)alkylenreste, wie der 4,4-Dicyclohexyl-2,2-propylenrest, der 4,4'-Dicyclohexylmethylen-Rest oder dessen alkylsubstituierten Homologen. Vorteilhaft eingesetzt werden können als Reste $R^1$ auch Heteroatome enthaltende Alkylenreste, wie beispielsweise der 3,6-Dioxa-1,8-octamethylen-, 3,6,9-Trioxa-1,11-undecamethylen- oder der 2,5,8-Trimethyl-3,6-dioxa-1,8-octamethylen-Rest und andere, die sich von Oligomeren des Ethylenoxids, Propylenoxids und ähnlichen ableiten. N-haltige Reste können beispielsweise sein der 3-(2-Ethylen)-3-aza-1,5-pentamethylen- oder der 3-Methyl-3-aza-1,5-pentamethylen-Rest, ferner solche, die sowohl Sauerstoff- als auch, vorzugsweise tertiäre, Stickstoffatome in der Alkylenkette enthalten. Der Rest $R^1$ kann weiterhin funktionelle Gruppen enthalten, wie beispielsweise Hydroxylgruppen, hydroxyalkyl-, hydroxycycloalkyl-, hydroxyoxaalkyl-und/oder hydroxyazaalkylsubstituierte, jeweils 2 bis 12 Kohlenstoffatome in der -alkyl-Gruppe enthaltende Harnstoff-, Carbamat-, Carbonamid- oder Sulfonamidgruppen.

Beispiele hierfür sind der 3-[2-(N-2-Ethyl-N'-butyl-N'-2-hydroxiethylureido]-3-aza-1,5-pentamethylen-, der 3-[-(-N'-2-Hydroxiethoxiethyl)-N-2-ethylureido]-3-aza-1,5-pentamethylen-oder der 4-[-2-(4-Hydroxibutyramido-N-2-ethyl-]-4-aza-1,7-hepatamethylen-Rest.

Die Reste $R^2$, $R^3$, $R^4$, $R^5$ und $R^6$ können untereinander gleich oder verschieden sein und für Wasserstoff, Alkylreste mit 1 bis 18 Kohlenstoffatomen, Cycloalkylreste mit 5 bis 15 Kohlenstoffatomen, Arylreste mit 6 bis 15 Kohlenstoffatomen, Aralkylreste mit 7 bis 16 Kohlenstoffatomen, Oxaalkyl- oder Azaalkylreste mit 2 bis 18 Kohlenstoffatomen, stehen, mit der Maßgabe, daß für den Fall, daß $R^2$ für einen Hydroxyalkylrest steht, dieser 4 bis 18 Kohlenstoffatome enthält, und $R^2$ nicht für einen Aryl- oder Aralkylrest steht. Die Strukturen der allgemeinen Formel (I) und/oder (II) können somit Propylenharnstoffeinheiten gleichen oder unterschiedlichen Substitutionsmusters enthalten. Für $R^2$ kann dabei kein aromatischer Rest wie Phenyl-, substituiertes Phenyl- etc. stehen, ebenso kein Aralkylrest wie beispielsweise der Benzylrest. Sonst kann seine Bedeutung die der Reste $R^3$ bis $R^6$ annehmen, also Wasserstoff, ein geradkettiger oder verzweigter Alkylrest sein, wie z.B. Methyl-, Ethyl-, n- und i-Propyl-, n- oder i-Butyl-, n-Pentyl-, n-Hexyl- oder 2-Ethylhexyl-Reste bedeuten.

$R^4$ und $R^5$ sollten dabei bevorzugt kurzkettige, unverzweigte Alkylreste, $R^6$ daneben auch bevorzugt Wasserstoffatome sein. Geeignet sind grundsätzlich jedoch auch Cycloalkylreste wie der Cyclohexylrest und seine alkylsubstituierten Homologen, Arylreste wie Phenyl-, Aralkylreste wie der Benzyl-, 2-Ethylphenyl-Rest und ähnliche. Die Substituenten $R^2$ bis $R^6$ können Sauerstoff- und/oder Stickstoffatome, vorzugsweise tertiäre Stickstoffatome, enthalten.

Beispiele hierfür sind der 1- oder 2-Methoxiethyl-, 2-Methoxipropyl-1- oder der N,N-Dimethylaminoethyl-Rest. Die Reste $R^2$ bis $R^6$ können ferner Hydroxylgruppen enthalten, wobei der Rest $R^2$ jedoch mindestens 4 Kohlenstoffatome aufweisen muß. Ein Beispiel hierfür ist der 2-Hydroxiethoxiethyl-Rest. Für $R^3$ bis $R^6$ sind jedoch ebenso geeignet der 2-Hydroxiethyl-, 2- bzw. 3-Hydroxipropyl- oder der N,N-Dimethyl-4-hydroxiethoxiaminoethyl-Rest und ähnliche.

Die erfindungsgemäßen Kondensationsprodukte können also auf verschiedene Weise hergestellt werden. So werden als Ausgangsmaterialien im allgemeinen Di- und/oder Polyharnstoffe eingesetzt, deren Synthese grundsätzlich in der Literatur beschrieben ist. Beispiele hierfür finden sich in Houben-Weyl, Bd. VIII, S. 149 ff.

So werden z.B. durch Umsetzung geeigneter primärer Di- und/oder Polyamine, deren Strukturen durch

den Rest $R^1$ definiert sind, mit Harnstoffen die entsprechenden Di- und/oder Polyharnstoffe erhalten. Das betreffende Amin kann dabei mit Harnstoff selbst, mono- und/oder symmetrisch disubstituierten Harnstoffen mit $C_1$- bis $C_4$-Alkylsubstituenten (z.B. Butylharnstoff oder N,N'-Dimethylharnstoff) bei Temperaturen von 100 bis 200 °C, vorzugsweise 120 bis 170 °C während 2 bis 18 Stunden, vorzugsweise 4 bis 8 Stunden zur Umsetzung gebracht werden. Pro primäre Aminogruppe des Amins werden dabei im allgemeinen 1,0 bis 1,5 Mol des betreffenden Harnstoffs eingesetzt. Größerer molarer Überschuß erfordert eine Isolierung des Polyharnstoffs vor der nachfolgenden Umsetzung und sollte daher vermieden werden.

Die Substituenten des eingesetzten Harnstoffs ergeben dann den oder die Substituenten $R^2$ der erfindungsgemäßen Kondensationsprodukte. In Gegenwart der in Anspruch 3 genannten Hydroxylverbindungen ergibt die Reaktion Di- und/oder Polyharnstoffe, die zusätzlich weitere, beispielsweise mit Hydroxialkylgruppen substituierte Harnstoffeinheiten oder auch hydroxylgruppentragende Carbonamid-, Carbamat- oder Sulfonamidgruppierungen aufweisen. Dadurch kann Aufbau von Molekulargewicht oder auch der Einbau von zu weiteren Umsetzung befähigten funktionellen Gruppen erreicht werden. Die beschriebene Bildung von Di- und/oder Polyharnstoffen kann in Substanz oder in Gegenwart inerter Lösungsmittel, beispielsweise Xylol, gegebenenfalls unter vermindertem oder erhöhtem Druck durchgeführt werden.

Die genannten Di- und/oder Polyharnstoffe können in bekannter Weise auch durch Umsetzung von Monoisocyanaten mit primären Di- und/oder Polyaminen bzw. Di- und/oder Polyisocyanaten mit primären Aminen erhalten werden. So werden mit Aminen, denen der Rest $R^1$ und Isocyanaten, denen der Rest $R^2$ zugrundeliegt, oder entsprechend umgekehrt, Produkte erhalten, die weitgehend denen aus dem vorher beschriebenen Herstellverfahren entsprechen. Die Reaktion wird dabei bevorzugt in wäßrigem Milieu bei Temperaturen von 0 bis 120 °C, vorzugsweise 20 bis 80 °C während 0,5 bis 6 Stunden, vorzugsweise 1 bis 3 Stunden, durchgeführt. Pro Mol primärer Amingruppe werden dabei im allgemeinen 0,8 bis 1,1 Mol Isocyanatgruppen zur Umsetzung gebracht. Die entstehenden Di- und/oder Polyharnstoffe fallen dabei in der Regel als relativ reine, kristalline Festkörper aus dem Reaktionsgemisch aus und können gegebenenfalls durch Filtration abgetrennt werden, während die nach vorgenanntem Verfahren erhaltenen Produkte überwiegend harzartigen Charakter haben.

Die nach beiden Verfahren gewonnenen Produkte können auch ohne weitere Reinigung im Eintopfverfahren weiter umgesetzt werden. Die Umsetzung erfolgt dabei mit 1,4 bis 2,5 Mol eines oder mehrerer Aldehyde pro Mol eingesetzter Harnstoffeinheit, wobei jedoch mindestens 0,7 Mol eines oder mehrerer Aldehyde verwendet werden müssen, die mindestens ein acides, zur Aldehydgruppe $\alpha$-ständiges Wasserstoffatom aufweisen. Beispiele hierfür sind Acetaldehyd, Propionaldehyd, Butyraldehyd, Isobutyraldehyd, 2-Methylbutanal und Phenylacetaldehyd.

Das Substitutionsmuster dieser Aldehydkomponente entspricht den beiden Resten $R^4$, $R^5$ und gegebenenfalls $R^6$. Eine gegebenenfalls weitere Aldehydkomponente, die dann den Rest $R^6$ ergibt, ist weitgehend frei wählbar und kann darüber hinaus z.B. Formaldehyd, Pivalaldehyd, Hydroxipivalaldehyd, Benzaldehyd, Furfurylaldehyd, Thiophenaldehyd und dergleichen sein.

Bevorzugt wird dabei die Verwendung von Isobutyraldehyd oder einer Mischung aus Formaldehyd und Isobutyraldehyd. Von den genannten Aldehyden können ebenso deren Acetale mit $C_1$-$C_6$-Alkoholen, Glykolen, wie Ethylenglykol, 1,2-Propylenglykol, 1,3-Propandiol und anderen eingesetzt werden, sofern die Reaktion in bevorzugten wäßrigen oder $C_1$-$C_6$-alkoholischem Medium durchgeführt wird. Als Lösungsmittel können jedoch auch inerte Lösungsmittel dienen; ferner ist die Verwendung flüssiger organischer Carbonsäuren, wie Ameisensäure oder Essigsäure vorteilhaft. Die Cyclokondensation der genannten Di- und/oder Polyharnstoffe mit den beschriebenen Aldehyden findet in Gegenwart saurer Katalysatoren, wie beispielsweise Mineralsäuren wie Salzsäure, Schwefelsäure, Phosphorsäure oder starker organischer Säuren, wie Ameisensäure oder Oxalsäure statt. Geeignete Katalysatoren sind ferner saure Ionenaustauscher. Man geht dabei zweckmäßigerweise so vor, daß man die Lösung oder Suspension der Harnstoff- und Aldehydkomponente auf Rückflußtemperatur, höchstens aber 80 °C erhitzt, und anschließend die Säure portionsweise zugibt. Es ist jedoch ebenso möglich, die Harnstoffkomponente, sauren Katalysator und gegebenenfalls die schwererflüchtige Aldehydkomponente vorzulegen, und anschließend bei Rückflußtemperaturen, höchstens aber 80 °C die niedriger siedende Aldehydkomponente oder das Aldehydgemisch portionsweise zuzugeben.

Die Reaktion ist in der Regel exotherm und wird bis zur vollständigen Umsetzung der Aldehyde, vorteilhaft bei der sich einstellenden Siedetemperatur, höchstens aber bei 150 °C durchgeführt. Bevorzugt ist der Temperaturbereich von 60 bis 120 °C. Die erforderliche Reaktionszeit liegt dabei zwischen 0,5 und 4 Stunden.

Die Reaktion ist gegebenenfalls auch unter Druck bis zu ca. 5 bar durchführbar.

Wird die Reaktion in wäßrigem Medium oder ohne Entfernung des Reaktionswassers durchgeführt, so bedeutet der Rest $R^3$ ein Wasserstoffatom. In Anwesenheit eines Alkohols, dessen Rest die genannte Bedeutung von $R^3$ hat, kann bei Entfernung des Reaktionswassers das betreffende Halbacetal hergestellt

werden.

Zur Aufarbeitung wird die zur Katalyse verwendete Säure in üblicher Weise neutralisiert, extrahiert, abdestilliert oder gegebenenfalls abfiltriert. Die gebildeten Di- und/oder Polypropylenharnstoffe fallen dabei als hellgelbe, harzartige Produkte an, die in manchen Fällen in Wasser, in anderen Fällen in aromatischen Kohlenwasserstoffen, wie Toluol oder Xylol, in den meisten Fällen in Alkoholen löslich sind, und so beispielsweise in butanolischer Lösung gewonnen und gereinigt werden können. Der am Ende eingestellte Festgehalt der betreffenden Harzlösung richtet sich nach dem vorgesehenen Verwendungszweck und der Viskosität der Produkte und beträgt in den meisten Fällen 60 bis 80 Gew.%.

Im Falle der Umsetzung der Kondensationsprodukte mit Di- und/oder Polyisocyanaten werden die substituierten Propylenharnstoffe, die Strukturen der genannten Formeln (I) und/oder (II) enthalten, über freie Hydroxylgruppen in den Resten $R^1$ und/oder $R^2$ mit Di- und/oder Polyisocyanaten unter Aufbau von Urethan-Struktureinheiten verknüpft, wobei vorzugsweise pro Mol Propylenharnstoff-Kondensationsprodukt der Formel (I) und/oder (II) 0,8 bis 1,2 Mol Isocyanatgruppen verwendet werden. Auf diese Weise werden verbrückte Strukturen erhalten, wobei als Brückenglieder die Reste der eingesetzten Di und/oder Polyisocyanate fungieren. Diese Reste können beispielsweise bedeuten Tetramethylen-, Hexamethylen-, Isophoron-, Diphenylmethan-, Toluylen-, Naphtylen- und ähnliche. Weiterhin sind geeignet für die Umsetzung mit den genannten Propylenharnstoffen Di- und/oder Polyisocyanate, die aus den oben genannten Diisocyanten durch Dimerisierung (z.B. über Urethdionbildung), Trimerisierung (z.B. über Isocyanuratbildung) oder Umsetzung mit aliphatischen Diolen oder gegebenenfalls höherwertigen Alkoholen, wie Ethylenglykol, Diethylenglykol, Propandiol, Butandiol, Hexandiol, Neopentylglykol, Trimethylolpropan, Glycerin, Pentaerythrit, Trimethylolbenzol und dergleichen gebildet werden können.

Die Kondensationsprodukte werden im allgemeinen in einem gegenüber Isocyanatgruppen inerten organischen Lösungsmittel, wie Chloralkanen, aromatischen Kohlenwasserstoffen, Ketonen, Estern oder deren Gemischen gelöst, gegebenenfalls noch vorhandenes Wasser oder Alkohol abdestilliert und anschließend das Di- oder Polyisocyanat zugegeben. Durch Kühlung wird die zunächst exotherm verlaufende Reaktion so kontrolliert, daß die Reaktionsmischung eine Temperatur von höchstens 50 ° C aufweist. Nach beendeter Isocyanatzugabe wird 0,5 bis 2 Stunden auf 60 bis 80 ° C erhitzt, bis vollständige Umsetzung des Isocyanats stattgefunden hat.

Das verwendete inerte Lösungsmittel kann wenn erforderlich, abdestilliert werden, wobei hellgelbe harzartige Produkte zurückbleiben, die dann in geeigneten Lösungsmitteln, wie Alkoholen oder aromatischen Kohlenwasserstoffen aufgenommen und gereinigt werden können. Der am Ende eingestellte Festgehalt der betreffenden Harzlösung richtet sich nach dem vorgesehenen Verwendungszweck und der Viskosität der Produkte und beträgt in den meisten Fällen 60 bis 80 Gew.%.

Im Falle der Umsetzung der Kondensationsprodukte mit Derivaten von Di- und/oder Polycarbonsäuren werden die substituierten Propylenharnstoffe der genannten Formel (I) und/oder (II) über freie Hydroxylgruppen in den Resten $R^1$ und/oder $R^2$ mit reaktiven Derivaten von Di- und/oder Polycarbonsäuren unter Esterbildung verknüpft, wobei pro Mol eingesetzten Harnstoffs b) 0,25 bis 1,0 Mol vorzugsweise Säurehalogenid- und/oder -anhydridgruppen eingesetzt werden. Auf diese Weise werden verbrückte Strukturen aufgebaut, wobei als Brückenglieder die Reste der eingesetzten Di- und/oder Polycarbonsäurederivate fungieren. Eingesetzt werden können beispielsweise Halogenide, wie Chloride und Bromide und/oder Anhydride von aliphatischen und/oder aromatischen Di- und/oder Polycarbonsäuren, wie beispielsweise Bernsteinsäure, Glutarsäure, Adipinsäure, Sebacinsäure, Pimelinsäure, Korksäure, dimerer Fettsäuren, Terephthalsäure, Phthalsäure, Trimellithsäure oder 1,2,4,5-Benzoltetracarbonsäure.

Zur weiteren Veresterung der im Propylenharnstoffharz gebundenen alkoholischen Hydroxylgruppen werden die gebildeten Di- und/oder Polypropylenharnstoffe zweckmäßigerweise möglichst wasser- und alkoholfrei in einem inerten Lösungsmittel unter Zugabe von Katalysator mit dem betreffenden Säurehalogenid, vorzugsweise Säurechlorid oder Säureanhydrid zur Umsetzung gebracht. Besonders bevorzugt wird die Veresterung mit Di- und/oder 1,2,4,5-Benzoltetracarbonsäuredianhydrid in essigsaurer Lösung. Die Umsetzung erfolgt dabei während 2 bis 4 Stunden bei Temperaturen von 60 bis 100 ° C. Nach beendeter Reaktion wird das Lösungsmitel entfernt und der Rückstand in beispielsweise butanolische Lösung überführt, in bekannter Weise säure- und salzfrei gewaschen und auf den gewünschten Festharzanteil gebracht. Der am Ende eingestellte Festgehalt der betreffenden Harzlösung richtet sich nach dem vorgesehenen Verwendungszweck und der Viskosität der Produkte und beträgt in den meisten Fällen 60 bis 80 Gew.%.

Im Falle der Umsetzung der Kondensationsprodukte mit p-substituierten Phenolen und/oder deren o-Methylolderivaten werden die substituierten Propylenharnstoffe der genannten Formeln (I) und/oder (II) über freie Hydroxylgruppen in den Resten $R^1$ und $R^2$ mit o-Methylolderivaten p-substituierter Phenole unter Aufbau von Methylenethergruppen, oder über das Stickstoffatom von Propylenharnstoffeinheiten mit $R^2$ gleich Wasserstoff mit o-Methylolphenolen unter Aufbau von N-Methylen-C-Gruppierungen, oder über eine

C-C-Bindung des $C_4$-Kohlenstoffatoms von Propylenharnstoffeinheiten mit phenolischen o-C-Atomen verknüpft. Auf diese Weise werden verbrückte Strukturen erhalten, wobei als Brückenglieder die Reste von p-substituierten Phenolen und/oder deren o-Methylolderivate fungieren. Beispiele für eingesetzte Phenole sind p-Alkylphenole, wie p-Kresol, p-Ethylphenol, p-Octylphenol, p-Nonylphenol, 4,4'-Dihydroxidiphenylmethan, Bisphenol-A und analoge sowie deren o-Methylolderivate, die vorzugsweise einen möglichst hohen o-Methylolierungsgrad aufweisen.

Die zur Katalyse verwendete Säure wird teilneutralisiert und zur weiteren Umsetzung das entsprechende Phenol oder o-Methylolphenol und ein Lösungsmittel zugesetzt, das mit Wasser azeotrope Gemische bildet, vorzugsweise Toluol, Xylol und insbesondere n- oder i-Butanol. Vorhandenes Restwasser sowie Reaktionswasser wird zweckmäßigerweise azeotrop abdestilliert, wobei ein pH-Wert von 1,5 bis 6, vorzugsweise 2,5 bis 4,5 und eine Temperatur von 60 bis 120, vorzugsweise 70 bis 100° C eingehalten wird. Nach Entfernung des Wassers wird in üblicher Weise neutralisiert, salzfrei gewaschen und die Harzlösung aufkonzentriert.

Die Menge des Phenols oder o-Methylolphenols wird so gewählt, daß pro Mol eingesetzten Harnstoffs b) o,25 bis 1 Mol reaktionsfähiger phenolischer o-Position oder o-Methylolgruppe verwendet werden.

Der am Ende eingestellte Festgehalt der betreffenden Harzlösung richtet sich nach dem vorgesehenen Verwendungszweck und der Viskosität der Produkte und beträgt in den meisten Fällen 60 bis 80 Gew.%.

Bei Verwendung der erfindungsgemäßen Kondensationsprodukte oder deren Umsetzungsprodukte als Komponente in hitzehärtbaren Überzugsmitteln, wird als Bindemittel ein Gemisch aus

(A) mindestens einem Polyadditions-, Polykondensations- oder Polymerisationsprodukt mit einem mittleren Molekulargewicht $\overline{M}_n$ von 500 bis 10 000, welches im Mittel pro Molekül mindestens zwei Hydroxylgruppen sowie zusätzlich gegebenenfalls primäre und/oder sekundäre und/oder tertiäre Aminogruppen besitzt und

(B) einem Kondensationsprodukt nach Anspruch 2 oder einem Umsetzungsprodukt nach Anspruch 1 eingesetzt.

Diese Überzugsmittel lassen sich für lösungsmittelhaltige oder wäßrige Einbrennlacke sowie zur Herstellung kathodisch abscheidbarer Elektrotauchlacke verwenden.

Die Verwendung der erfindungsgemäßen Kondensationsprodukte in hitzehärtbaren Überzugsmittel erfolgt in der Weise, daß sie als Komponente (B) mit einer Bindemittelkomponente (A), gegebenenfalls mit einem geeigneten Lösungsmittel gemischt wird.

Die Komponente (A) kann dabei ein Polyadditions-, Polykondensations- oder Polymerisationsprodukt mit einem mittleren Molekulargewich $\overline{M}_n$ von 500 bis 10 000 und aus den verschiedensten Verbindungsklassen ausgewählt sein. Wichtig ist allein, daß sie im Mittel zwei OH- und gegebenenfalls primäre und/oder sekundäre und/oder tertiäre Aminogruppen besitzt. Beispiele geeigneter Materialien sind Polyester, Alkydharze, Polyether, Polyacrylatharze, Polyurethane, Epoxidharze und ihre Umsetzungsprodukte mit Alkoholen, Mercaptanen oder Aminen. Eine weitere geeignete Verbindungsklasse sind Polydienharze oder -öle, z.B. Polybutadienöle. In diese lassen sich z.B. durch Addition von Mercaptoethanol an einen Teil der Doppelbindungen OH-Gruppen einführen. Eine weitere Möglichkeit, OH-Gruppen einzuführen, ist die Umsetzung mit Maleinsäureanhydrid und nachfolgende Reaktion mit OH-haltigen Aminen wie Ethanolamin oder Diethanolamin. Auch durch Epoxidierung der Polybutadienöle mit Persäuren und anschließende Umsetzung mit Aminen läßt sich die benötigte Derivatisierung durchführen.

Geeignete Polyester sind solche mit einem mittleren Molekulargewicht $\overline{M}_n$ von 500 bis 10 000 und einer Hydroxylzahl von 25 bis 400 aus aliphatischen und/oder aromatischen Dicarbonsäuren mit 4 bis 10 Kohlenstoffatomen, beispielsweise Bernsteinsäure, Glutarsäure, Adipinsäure, Pimelinsäure, Korksäure, Azelainsäure, Sebacinsäure, Cyclohexandicarbonsäure, Phthalsäure, Isophthalsäure oder Terephthalsäure bzw. deren Derivaten und mehrwertigen Alkoholen, wie aliphatischen Diolen, wie Ethylenglykol, Diethylenglykol, Triethylenglykol, Polyethylenglykol, Propandiol, Butandiol, Hexandiol, Neopentylglykol oder Hydroxypivalinsäureneopentylglykolester und gegebenenfalls höherwertigen Alkoholen, wie Trimethylolpropan, Glycerin, Erythrit, Pentaerythrit, Trimethylolbenzol oder Trishydroxyethylisocyanurat.

Geeignete Alkydharze sind ähnlich aufgebaut, nur enthalten sie eine der mehrere Monocarbonsäuren wie z.B. Fettsäuren. Auch Alkydharze, welche Glycidylester von verzweigten Carbonsäuren enthalten, können verwendet werden.

Geeignete Polyether sind z.B. aliphatische oder araliphatische Polyether, welche durch Umsetzung von zwei- und/oder mehrwertigen Alkoholen, mit verschiedenen Mengen an Ethylen- und/oder Propylenoxid erhalten werden.

Geeignete Polyacrylate sind OH-gruppenhaltige Polyacrylate mit einer Hydroxylzahl von 25 bis 500. Diese sollten eine Säurezahl < 25, bevorzugt < 10 und einen K-Wert nach Fikentscher (3 %ig in Aceton) von 10 bis 40, vorzugseise von 12 bis 25 aufweisen und können z.B. folgende Monomeren einpolymerisiert

enthalten:
10 bis 100 Gew.%, vorzugsweise 20 bis 40 Gew.%, mindestens eines OH- oder NH-Gruppen enthaltenden Monomeren, beispielsweise Isopropylaminopropylmethacrylamidoder Hydroxy-($C_2$-$C_4$)-alkylester einer $\alpha,\beta$-ethylenisch ungesättigten Carbonsäuren, beispielsweise 2-Hydroxyethyl- und Hydroxypropyl(meth)acrylat sowie Butandiol-mono(meth)acrylat, 0 bis 90 Gew.%, vorzugsweise 60 bis 80 Gew.%, mindestens einer ethylenisch ungesättigten Carboxyl- und hydroxylgruppenfreien Verbindung, beispielsweise Vinylaromaten, wie Styrol und Vinyltoluol, Vinylester von Carbonsäuren mit 2 bis 18 Kohlenstoffatomen, wie Vinylacetat und Vinylpropionat, Vinylether von Monoalkanolen mit 1 bis 18 Kohlenstoffen, wie Vinylmethylether und Vinylisobutylether, Ester der Acrylsäure oder Methacrylsäure mit $C_1$-$C_{12}$-Monoalkanolen, entsprechende Maleinsäure-, Fumarsäure- und Itaconsäurediester, (Meth-)acrylsäureamid, (Meth-)acrylsäurenitril, Monomere mit tertiären Aminogruppen, wie Diethylaminoethylacrylat oder Diethylaminoethylacrylamidsowie Gemische dieser Monomeren. Eine weitere Möglichkeit, basische Acrylate zu erhalten, ist der Einsatz von Epoxidgruppen tragenden Monomeren wie Glycidylmethacrylat und Addition von Aminen and die Oxiranringe der Polymerisate.

Geeignete Polyurethane sind:
OH-gruppenhaltige Polyurethane mit einer Hydroxylzahl von 25 bis 600 aus aliphatischen und/oder aromatischen Diisocyanaten, die beispielsweise aus Tetramethylendiisocyanat, Hexamethylendiisocyanat, Isophorondiisocyanat, Diphenylmethandiisocyanat, Toluylendiisocyanat, Naphthylendiisocyanat, 4,4'-Diphenyletherdiisocyanat, sowie gegebenenfalls daraus hervorgehende Di- oder Trimere, und aliphatischen Diolen, wie Ethylenglykol, Diethylenglykol, Triethylenglykol, Polyethylenglykole, Propandiol, Butandiol, Hexandiol, Neeopentylglykol oder Hydroxypivalinsäureneopentylglykolester und gegebenenfalls höherwertigen Alkoholen, wie Trimethylolpropan, Glycerin, Pentaerythrit, Trimethylolbenzol oder Trishydroxyethylisocyanurat erhältlich sind.

Geeignete Epoxidharze sind z.B. Glycidylether, wie sie aus 2,2-Bis-(4-hydroxyphenyl)-propan und Epichlorhydrin hergestellt werden. Diese Epoxidharze können weiter modifiziert sein, z.B. durch Umsetzung mit polyfunktionellen Alkoholen oder SH-Verbindungen. Beispiele solcher, für die Modifizierung geeigneter polyfunktioneller Alkohole sind Ethylenglykol, Propylenglykol-1,2, Propylenglykol-1,3 und Butandiol-1,4.

Ist eine Elastifizierung erwünscht, können auch noch langkettige polyfunktionelle Alkohole oder Mercaptane eingesetzt werden. Werden die polyfunktionellen Alkohole oder Mercaptane in größeren als äquivalenten Mengen in Bezug auf die vorhandenen Epoxidgruppen eingesetzt, so entstehen Produkte mit endständigen OH- oder SH-Gruppen. Setzt man dagegen geringere als äquivalente Mengen ein, so entstehen Produkte mit endständigen Epoxidgruppen, die gegebenenfalls weiter umgesetzt werden können. Während die Umsetzung der Mercaptane mit Epoxidgruppen breits ohne Katalysator abläuft, ist für die Umsetzung der Alkohole die Verwendung eines Katalysators wie z.B. Dimethylbenzylamin und höherer Temperaturen von etwa 50 bis 150 ° C notwendig.

Umsetzungsprodukte von Epoxidharzen mit primären oder sekundären Aminen sind ebenfalls als Komponente (A) einsetzbar. Dabei bietet sich besonders die Umsetzung mit hydroxylgruppenhaltigen Aminen, wie z.B. Ethanolamin, Methylethanolamin und Diethanolamin an.

Werden als Komponente (A) solche Produkte eingesetzt, die ausreichende Mengen an Aminogruppen enthalten, um nach Protonierung mit Säuren wasserlöslich oder wasserdispergierbar zu werden, lassen sich in Kombination mit Komponente (B) wasserdispergierbare Einbrennlackbindemittel herstellen, insbesondere solche, die für die kathodische Elektrotauchlackierung verwendet werden können. Für diesen Zweck können die oben erwähnten Umsetzungsprodukte von Epoxidharzen mit primären oder sekundären Aminen eingesetzt werden.

Viele der für die kathodische Elektrotauchlackierung vorgeschlagenen Trägerharze sind auch als Komponente (A) in den erfindungsgemäßen Überzugsmitteln einsetzbar, so z.B. die Umsetzungsprodukte phenolischer Mannichbasen mit Epoxidharzen gemäß der DE-PS 24 19 179, die Umsetzungsprodukte von kettenverlängerten Epoxidharzen mit sekundären Aminen gemäß der US-PS 4 104 140, Umsetzungsprodukte von (Meth)-acrylamidomethylierten Phenolen, Aminen und Epoxidharzen, z.B. gemäß DE-OS 29 42 488 und DE-OS 30 21 300; wichtig ist nur, daß sie ein Molekulargewicht von 500 bis 10 000 aufweisen und im Mittel pro Molekül mindestens zwei OH- und primäre und/oder sekundäre Aminogruppen besitzen. Während es für die Vernetzungsaktivität beim Einbrennen völlig ausreicht, wenn die Komponente (A) lediglich OH- und keine primären und/oder sekundären Aminogruppen enthält, ist es doch häufig vorteilhaft, Produkte einzusetzen, die auch primäre und/oder sekundäre Aminogruppen enthalten, da sich damit wäßrige Elektrotauchlackbäder mit hohen pH-Werten von z.B. 6,5 bis 8,0 herstellen lassen. Durch hohe pH-Werte, vor allem solche nahe pH 7 oder darüber, läßt sich eine Korrosion von Anlagen vermeiden. Eine Möglichkeit, zu als Komponente (A) geeigneten Produkten mit primären und sekundären Aminogruppen zu kommen, ist die Umsetzung von überschüssigen primären Diaminen mit Epoxidharzen und anschließende

Abtrennung des überschüssigen Amins bei erhöhter Temperatur und vermindertem Druck.

Als Diamine kommen dafür vor allem solche mit 2 bis 6 Kohlenstoffatomen in Betracht, z.B. Ethylendiamin, 1,2- und 1,3-Diaminopropan, 1,4-Diaminobutan und Hexamethylendiamin. Die Reaktionsprodukte können, falls gewünscht, mit Dicarbonsäuren kettenverlängert werden, z.B. mit Sebacinsäure oder mit dimerer Fettsäure. Durch das Verhältnis Dicarbonsäure zu Epoxidharz-Aminaddukt kann man das gewünschte Molekulargewicht einstellen, z.B. kann man auf zwei Moleküle Epoxidharz-Aminaddukt 1 Mol dimerer Fettsäure einsetzen.

Eine weitere Möglichkeit, als Komponente (A) geeignete Produkte mit primären Aminogruppen herzustellen, ist der Umsatz von Epoxidharzen mit sekundären Aminen, die geblockte primäre Aminogruppen enthalten. Beispiele für solche Amine sind das Diketimin von Diethylentriamin, das Ketimin von Aminoethylethanolamin und das Ketimin von N-Methyl-ethylendiamin. Die Ketimine lassen sich in einfacher Weise aus den freien Aminen und einem Keton, z.B. Methylisobutylketon, unter Auskreisen von Wasser herstellen. Bei der Umsetzung mit Epoxidharzen reagiert nur die sekundäre Aminogruppe, anschließend kann das Ketimin durch Zugabe von Wasser gespalten werden, wobei sich die freie primäre Aminogruppe zurückbildet.

Durch Umsetzung eines Teils der primären Aminogruppen mit Dicarbonsäuren lassen sich auch diese Produkte durch Kettenverlängerung elastifizieren.

Komponente (A) ist im erfindungsgemäßen Bindemittelgemisch im allgemeinen in einer Menge von 30 bis 95, vorzugsweise 60 bis 85 Gew.%, der Gesamtmenge des Bindemittelgemisches enthalten.

Komponente (B) wird im allgemeinen in einer Menge von 5 bis 70, vorzugsweise 15 bis 40 Gew.%, bezogen auf die Gesamtmenge des Bindemittelgemisches (A) + (B), eingesetzt.

Zur Herstellung der erfindungsgemäßen Überzugsmittel werden die Komponenten (A) und (B) gemischt. Bei Komponenten mit niedriger Viskosität kann dies in Substanz erfolgen, wobei gegebenenfalls auf Temperaturen bis maximal 100 °C erwärmt wird. Produkte höherer Viskosität werden vor der Mischung in organischen Lösungsmitteln gelöst. Dabei können übliche Lösemittel wie Alkohole, Ketone, Ester, Ether, Kohlenwasserstoffe etc. verwendet werden.

Die erfindungsgemäßen Überzugsmittel können, gegebenenfalls mit Zusätzen von Pigmenten, Hilfsmitteln und Härtungskatalysatoren, durch übliche Methoden, wie Spritzen, Tauchen, Gießen und Rakeln auf Substrate wie Holz, Kunststoff oder Metall gebracht werden.

Produkte, welche, bedingt durch ihren Gehalt an Aminogruppen, nach Neutralisation mit Säuren, wie z.B. Essigsäure, wasserdispergierbar werden, können auch als wäßrige Dispersion angewandt werden. Derartige Produkte lassen sich vorteilhaft für die Elektrotauchlackierung elektrisch leitfähiger Substrate, wie z.B. Metallteilen, Blechen usw. aus Messing, Kupfer, Aluminium, metallisierter Kunststoffe oder mit leitendem Kohlenstoff überzogener Materialien, sowie Eisen und Stahl, die gegebenenfalls chemisch vorbehandelt, z.B. phosphatiert, sind, verwenden. Dazu wird zur zumindest teilweisen Neutralisation eine Säure, wie z.B. Ameisenäure, Essigsäure oder Milchsäure, eingerührt und mit Wasser auf die Verarbeitungskonzentration verdünnt.

Für die kathodische Elektrotauchlackierung wird im allgemeinen ein Feststoffgehalt von 5 bis 30 Gew.% eingestellt. Die Abscheidung erfolgt üblicherweise bei Temperaturen von 15 bis 40 °C während einer Zeit von 1 bis 5 Minuten und bei pH-Werten von 5,0 bis 9, vorzugsweise pH 6,5 bis 8,0, bei Abscheidespannungen von 50 bis 500 Volt. Der zu beschichtende elektrisch leitende Körper wird dabei als Kathode geschaltet. Der abgeschiedene Film wird bei Temperaturen oberhalb von 100 °C ca. 20 Minuten gehärtet.

Die folgenden Beispiele sollen die Erfindung näher erläutern, ohne sie zu beschränken. Die in den Beispielen genannten Teile und Prozente sind, soweit nicht anders angegeben, Gewichtsteile und Gewichtsprozente.

Der Übersicht halber sind jeweils nur die dem Strukturtyp (I) entsprechenden Formeln aufgeführt, obwohl in den beschriebenen Produkten naturgemäß die möglichen Isomeren des Strukturtyp (II) enthalten sind.

Beispiel 1

Herstellung eines Kondensationsproduktes, das im wesentlichen Strukturen der Formel

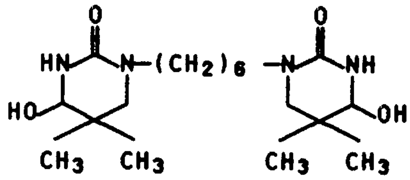

enthält:

1) 168 Teile Hexamethylendiisocyanat werden zu 350 Teilen einer 12 %igen wäßrigen Ammoniaklösung getropft. Die Temperatur wird bei 40 ° C gehalten bis Isocyanatzugabe abgeschlossen ist; anschließend wird 1 Stunde bei 80 ° C nachgerührt. Der ausgefallene Bisharnstoff wird abfiltriert und mit heißem Wasser neutralgewaschen, dann umkristallisiert, Ausbeute 132 Teile Hexamethylenbisharnstoff (65 % d.Th.), Schmelzpunkt 197-200 ° C.

2) Gemäß Houben-Weyl, Bd. VIII, S. 151 werden 115 Teile Hexamethylendiamin und 180 Teile Harnstoff 3 bis 4 Stunden auf 130 bis 140 ° C erhitzt und das sich verfestigende Reaktionsprodukt aus Wasser umkristallisiert.

101 Teile des nach 1) oder 2) erhaltenen Hexamethylendiharnstoffs werden mit 75 Teilen 40 %igen wäßrigen Formaldehyds, 72 Teilen Isobutyraldehyd und 100 Teilen Wasser unter Rühren auf Rückflußtemperatur (ca. 62 ° C) erhitzt und 20 Minuten am Rückfluß gehalten. Anschließend werden 72 Teile conc. Salzsäure so rasch zugetropft, daß lebhafter Rückfluß besteht. Die Temperatur steigt auf ca. 105 ° C an. Anschließend wird 2 Stunden bei Rückflußtemperatur gerührt. Nach Abkühlen auf 60 ° C wird mit Natronlauge pH 6 eingestellt, Wasser abgezogen und eine Mischung aus 66 Teilen Isobutanol und 50 Teilen Methanol zugegeben, die Mischung homogenisiert und Salz abfiltriert. Man erhält 252 Teile einer hellgelben, leicht viskosen Harzlösung von 57 % Festgehalt.

Beispiel 2

Herstellung eines Kondensationsproduktes, das im wesentlichen Strukturen der Formel

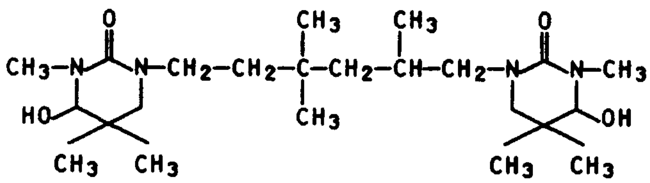

enthält.

158 Teile 2,4,4-Trimethylhexamethylendiamin-1,6 und 195 Teile N,N'-Dimethylharnstoff werden auf 120 ° C erhitzt und 2 Stunden bei dieser Temperatur gerührt. Dabei wird ständig Stickstoff über die Schmelze geleitet. Anschließend wird weitere 2 Stunden bei 160 ° C gerührt; dann werden 850 Teile Wasser zugesetzt und 30 Minuten bei 80 bis 100 ° C unter Stickstoffeinleitung gerührt. Nach Abkühlen auf 40 ° C wird das Wasser abdekantiert, 150 Teile 40 %iger wäßriger Formaldehyd und 144 Teile Isobutyraldehyd zugegeben und 15 Minuten bei Rückflußtemperatur (68 ° C) gerührt. Nach Zugabe von 150 Teilen conc. Salzsäure steigt die Rückflußtemperatur auf 104 ° C an. Bei dieser Temperatur wird 2 Stunden gerührt, mit Natronlauge neutralisiert und das Produkt mit Toluol extrahiert. Nach zweimaliger Extraktion der toluolischen Lösung mit Wasser und Aufkonzentrieren werden 372 Teile einer 79 %igen toluolischen gelbbraunen Harzlösung erhalten.

Beispiel 3

Herstellung eines Kondensationsproduktes, das im wesentlichen Strukturen der Formel

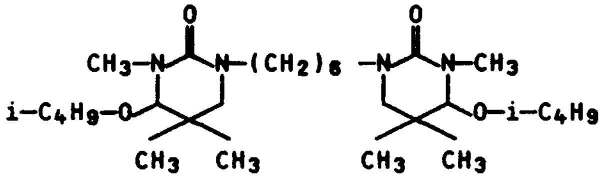

enthält:
67 Teile Hexamethylendiisocyanat werden in 62 Teilen einer 40 %igen wäßrigen Methylaminlösung und 200 Teilen Wasser getropft und nach beendeter Zugabe 1 Stunde bei 60 ° C gerührt. Der ausgefallene Harnstoff wird abgesaugt und 80 Teile davon in eine Mischung aus 50 Teilen Isobutyraldehyd und 76 Teilen einer 30 %igen wäßrigen Formaldehydlösung eingetragen. Die dicke Suspension wird auf 62 ° C aufgeheizt; dann werden innerhalb von 1 Minute 15 Teile conc. Salzsäure zugegeben.

Die Rückflußtemperatur steigt auf 102 ° C an und wird 90 Minuten gehalten. Dann wird mit Natronlauge auf pH 3-4 eingestellt und 580 Teile i-Butanol zugesetzt. Hierauf wird bei 70 bis 80 ° C bei schwachem Vakuum möglichst vollständig Wasser ausgekreist, anschließend mit Natronlauge auf pH 7 bis 7,5 neutralisiert und i-Butanol abgezogen bis zu einer Viskosität der Lösung von ca. 1500 mPa.s bei 30 ° C. Anschließend wird vom Salz abfiltriert. Man erhält 289 Teile einer 75 %igen i-butanolischen Harzlösung.

Beispiel 4

Herstellung eines Kondensationsproduktes, das im wesentlichen Strukturen der Formel

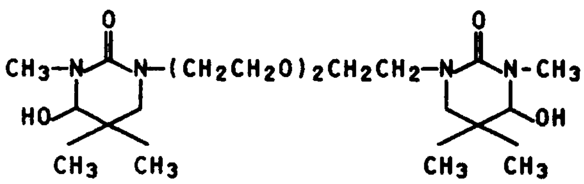

enthält:
148 Teile 3,6-Dioxa-octandiamin-1,8 und 193 Teile N,N'-Dimethylharnstoff werden unter Einleitung von Stickstoff je 1 Stunde bei 130 und 135 ° C, anschließend 2,5 Stunden bei 140 ° C gerührt, bis durch Austritt von ca. 60 Teilen Methylamin weitgehende Umsetzung erfolgt ist.

280 Teile des so erhaltenen Harnstoffs werden mit 160 Teilen 40 %igem wäßrigem Formaldehyd und 153 Teilen Isobutyraldehyd gemischt und auf 65 ° C aufgeheizt. Nach Zugabe von 42 Teilen conc. Salzsäure steigt die Rückflußtemperatur auf ca. 102 ° C an. Bei dieser Temperatur wird 90 min gerührt, anschließend mit Natronlauge neutralisiert, Wasser abdestilliert und der Rückstand mit Methanol aufgenommen. Nach Filtration von Salz und Abdestillieren von Methanol wird mit Wasser verdünnt bis auf einen Festgehalt von 50 %. Ausbeute: 840 Teile einer wäßrigen Harzlösung mit einer Viskosität von 13 mPa.s (20 ° C).

Beispiel 5

Herstellung eines Kondensationproduktes, das im wesentlichen Strukturen der Formel

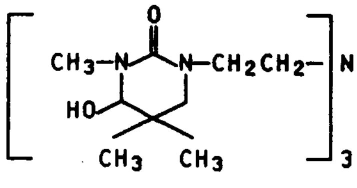

enthält:
292 Teile Tris-2-aminoethyl-amin und 528 Teile N,N'-Dimethylharnstoff werden je 1 Stunde bei 120 und 140 ° C, dann 2 Stunden bei 150 ° C und abschließend 0,5 Stunden bei 160 ° C unter Stickstoff gerührt. Die

Viskosität der Reaktionsmischung steigt gegen Ende der Reaktion deutlich an. Anschließend werden 350 Teile Wasser und 50 Teile Eisessig zugegeben und unter Stickstoff 1 Stunde am Rückfluß gerührt. Anschließend werden 450 Teile Formaldehyd (40 %ig in Wasser) und 144 Teile conc. Salpetersäure zugesetzt und auf 65° C erhitzt. Dann werden 432 Teile Isobutyraldehyd so rasch zugetropft, daß die Reaktionsmischung am lebhaften Rückfluß bleibt. Die Rückflußtemperatur steigt auf ca. 103° C an, und die Mischung wird 2 Stunden bei dieser Temperatur gerührt. Nach Abkühlen wird mit Natronlauge pH 8,1 eingestellt und 800 Teile Butanol und 120 Teile Methanol zugegeben. Nach Abtrennen der wäßrigen Phase, Waschen der organischen Phase mit Wasser und Aufkonzentrieren im Vakuum werden 1254 Teile einer 74,5 %igen Harzlösung erhalten.

Beispiel 6

Herstellung eines Kondensationsproduktes, das im wesentlichen Strukturen der Formel

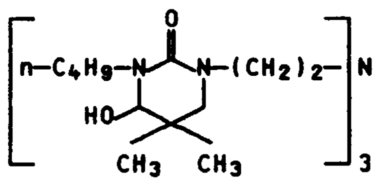

enthält:
49 Teile Tris-(2-aminoethyl)-amin werden in 200 Teilen Aceton gelöst und in eine MIschung aus 99 Teilen n-Butylisocyanat in 200 Teilen Aceton getropft. Durch Eiskühlung wird die Temperatur zunächst bei 40° C gehalten und nach beendeter exothermer Reaktion für 30 Minuten auf 60° C gebracht. Nach Zugabe von 200 Teilen Wasser werden weitere 30 Minuten bei 80° C gerührt, anschließend der Trisharnstoff abgesaugt, mit Wasser gewaschen und getrocknet. Ausbeute: 114 Teile (77 % d.Th.). Das Produkt wird in 200 Teilen Wasser, 50 Teilen Eisessig und 58 Teilen Formaldehyd (40 %ig in Wasser) aufgenommen. Nach Zugabe von 45 Teilen conc. Salpetersäure entsteht eine klare Lösung, die auf ca. 60° C erhitzt wird. Während des Zutropfens von 55 Teilen Isobutyraldehyd steigt die Rückflußtemperatur auf ca. 100° C an. Nach 1 Stunde Rühren bei Rückflußtemperatur wird abgekühlt, mit Natronlauge pH 7 eingestellt und das Produkt in 350 Teilen Toluol aufgenommen. Nach zweimaligem Waschen mit Wasser wird die organische Phase auf einem Festharzgehalt von 75 % aufkonzentriert (Viskosität ca. 2000 mPas).

Beispiel 7

Herstellung eines Kondensationsproduktes, das im wesentlichen Strukturen der Formel

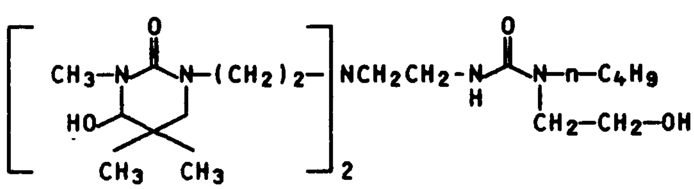

enthält:
292 Teile Tris-(2-aminoethyl)-amin, 120 Teile Harnstoff und 352 Teile N,N'-Dimethylharnstoff werden während 3,5 Stunden kontinuierlich von 120 auf 160° C unter Einleiten von Stickstoff erhitzt. Anschließend werden 214 Teile n-Butylethanolamin eingetragen und weitere 3 Stunden bei 140 bis 160° C gerührt.

Der Rest flüchtiger Anteile wird bei 140° C im Vakuum abgezogen. Anschließend werden 400 Teile Wasser, 100 Teile Eisessig, 294 Teile Formaldehyd (40 %ig in Wasser) und 283 Teile Isobutyraldehyd zugegeben und auf Rückflußtemperatur erhitzt (ca. 62° C). Dann werden 230 Teile conc. Salpetersäure zugetropft, wobei die Rückflußtemperatur auf 103° C steigt. Bei dieser Temperatur wird 2 Stunden gerührt. Nach Abkühlen und Neutralisation mit Natronlauge werden 1200 Teile i-Butanol zugegeben und die organische Phase abgetrennt, mit Wasser gewaschen und auf 1140 Teile einer 61,5 %igen Harzlösung aufkonzentriert.

Beispiel 8

Herstellung eines Kondensationsproduktes, das im wesentlichen Strukturen der Formel

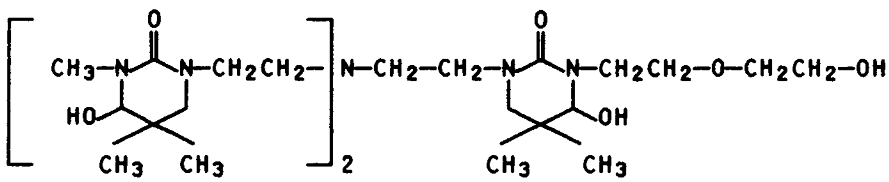

enthält:
176 Teile N,N'-Dimethylharnstoff, 60 Teile Harnstoff und 146 Teile Tris-2-aminoethyl-amin werden innerhalb von 3,5 Stunden von 120 auf 160°C erhitzt, anschließend 105 Teile 2,2-Aminoethoxiethanol zugesetzt und weitere 3 Stunden bei Temperaturen von 140 bis 160°C ansteigend erhitzt. Dann werden 200 Teile Wasser und 50 Teile Eisessig zugesetzt. Nach Zugabe von 225 Teilen 40 %iger wäßriger Formaldehydlösung und 216 Teilen Isobutyraldehyd wird auf Rückflußtemperatur erwärmt und innerhalb von 20 Minuten 150 Teile conc. Salpetersäure zugegeben. Die Rückflußtemperatur steigt dabei auf ca. 100°C an. Bei dieser Temperatur wird zwei Stunden lang gerührt, abgekühlt und mit Natronlauge neutralisiert. Zur Entfernung des Salzes wird das Reaktionsgemisch aufkonzentriert, der Rückstand in Methanol aufgenommen, filtriert, erneut aufkonzentriert und das hellgelbe harzartige Produkt in Wasser aufgenommen. Aubeute: 924 Teile (50 %ig in Wasser).

Beispiel 9

Herstellung eines Kondensationsproduktes, das im wesentlichen Strukturen der Formel

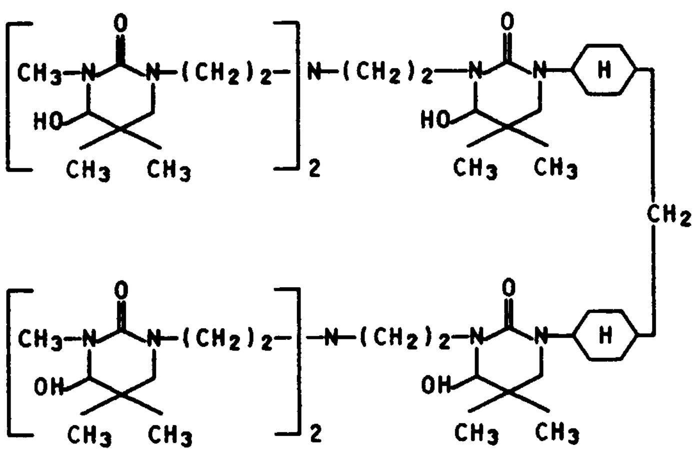

enthält:
264 Teile N,N'-Dimethylharnstoff, 146 Teile Tris-2-aminoethylamin und 101 Teile 4,4'-Diaminodicyclohexylmethan werden innerhalb von 5,5 Stunden von 80 auf 160°C erhitzt, wobei Stickstoff über die Schmelze geleitet wird. 125 Teile Wasser und 125 Teile Eisessig werden rasch zugegeben und 1 Stunde am Rückfluß gerührt. Anschließend werden 214 Teile 40 %iger wäßriger Formaldehyd und 205 Teile Isobutyraldehyd zugesetzt und auf 65°C erhitzt. 210 Teile conc. Salpetersäure werden möglichst rasch zugesetzt. Die zunächst viskose Reaktionsmischung wird dabei wieder dünnflüssig und die Rückflußtemperatur steigt auf 104°C. Bei dieser Temperatur wird 2 Stunden gerührt. Mit Natronlauge wird der pH auf 6,5 eingestellt und mit 2000 Teilen i-Butanol das Harz extrahiert. Die i-butanolische Harzlösung wird mit Wasser gewaschen und auf 63,5 % Festgehalt aufkonzentriert. Ausbeute 642 Teile.

Beispiel 10

Herstellung eines Kondensationsproduktes, das im wesentlichen Strukturen der Formel

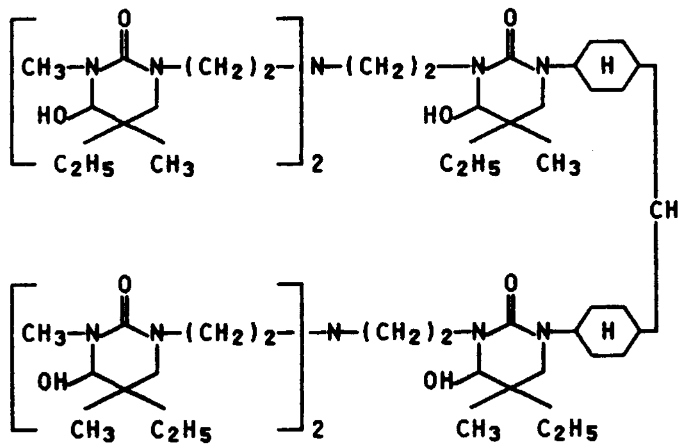

enthält:
Man verfährt wie in Beispiel 9 angegeben, nur werden 205 Teile Isobutyraldehyd durch 245 Teile 2-Methylbutanal ersetzt. Ausbeute: 648 Teile einer 64,5 %igen Harzlösung in i-Butanol.

Beispiel 11

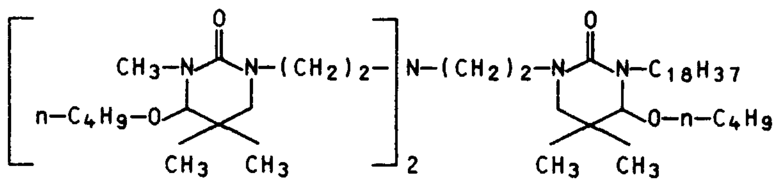

292 Teile Tris-2-aminoethylamin, 528 Teile N,N'-Dimethylharnstoff und 538 Teile Stearylamin werden in 5 Stunden von 120 auf 160 ° C erhitzt und unter Stickstoff gerührt. Anschließend werden 2000 Teile Butanol und 500 Teile conc. Salzsäure zugegeben und die Mischung auf ca. 60 ° C gebracht. Nach Zugabe von 180 Teilen Paraformaldehyd wird die Mischung 15 Minuten bei 60 ° C gerührt, anschließend werden 432 Teile Isobutyraldehyd so zugetropft, daß die Mischung am lebhaften Rückfluß siedet. Die Temperatur steigt dabei auf 100 bis 102 ° C an und die Reaktionsmischung wird klar. Nach 2 Stunden Rückfluß wird mit Natronlauge neutralisiert, vom Salz abfiltriert, die butanolische Phase 2-mal mit Wasser gewaschen und die organische Phase aufdestilliert bis zu einem Festgehalt von 55 %. Ausbeute: 640 Teile einer klaren schwach gelblichen Harzlösung.

Anwendungsbeispiel 1

Eine Lackformulierung wurde hergestellt aus
70 Teilen (bezogen auf fest) eines der folgenden Lackbindemittel A1 bis A3
und
30 Teilen (bezogen auf fest) eines der erfindungsgemäßen Kondensationsprodukte B1 bis B4 gemäß den Beispielen 1, 5, 7 oder 9, bzw. zum Vergleich handelsüblichen Produkten B5 bis B8.

Die Mischung der beiden Komponenten wurde mit Ethanol auf ca. 50 bis 60 % eingestellt und auf ein Stahlblech mit einem 100 µm Rakel aufgezogen.

Anschließend werden die Lacke 20 Minuten bei den in der Tabelle angegebenen Temperaturen eingebrannt. Die Prüfung der Lackfilme erfolgte durch 100 maliges Reiben mit einem mit Aceton getränkten Wattebauschs, die Ergebnisse sind in Tabelle I wiedergegeben.

A1 Handelsübliches Lackbindemittel auf Polyesterbasis (z.B. Alkydal® R40)

A2 Nicht selbstvernetzendes, kathodisch abscheidbares Bindemittel, hergestellt durch Umsetzung von 400 Teilen Hexamethylendiamin und 400 Teilen einer 80 %igen Lösung eines handelsüblichen Epoxidharzes auf Basis von 2,2-Bis-(4-hydroxiphenyl)-propan in Toluol bei 80 bis 100 ° C und Abdestillieren von Toluol und überschüssigem Amin bis zu einer Aminzahl von 160 mg

KOH/g und einem Erweichungspunkt von ca. 100 °C, und anschließender Umsetzung von 400 Teilen dieses Addukts mit 140 Teilen Dimerfettsäure, 17,5 Teilen Stearinsäure, 59 Teilen Phenyldiglycol, 37 Teilen Benzylalkohol, 9 Teilen Triphenylphosphin, 43 Teilen Toluol und 12 Teilen Ethylendiamin bei 170 °C unter Abdestillieren von Wasser bis zu einer Säurezahl von 4 bis 8 und anschließender Einstellung eines Festgehaltes von 70 % mit Butylglycol, Ethanol und Wasser im Massenverhältnis 7:5:6.

A3 OH-gruppenhaltiges Polyacrylat, hergestellt durch Copolymerisation von 40 Teilen Butylacrylat, 20 Teilen Styrol, 20 Teilen Methylmethacrylat, 15 Teilen Hydroxipropylacrylat und 5 Teilen Acrylsäure mit einem Festgehalt von 26 % in Wasser, einem K-Wert von 25,7 und einer Säurezahl von 42 bei einem Neutralisationsgrad von 70 % mit Dimethylethanolamin.

B1 Erfindungsgemäßes Kondensationsprodukt gemäß Beispiel 1.

B2 Erfindungsgemäßes Kondensationsprodukt gemäß Beispiel 5.

B3 Erfindungsgemäßes Kondensationsprodukt gemäß Beispiel 7.

B4 Erfindungsgemäßes Kondensationsprodukt gemäß Beispiel 9.

Zum Vergleich

B5 Handelsübliches Melamin-Formaldheyd-Lackharz, butanolverethert (z.B. Luwipal®015).

B6 Handelsübliches Harnstoff-Formaldehyd-Lackharz, isobutanolverethert (z.B. Plastopal® FIB).

B7 Vernetzer auf Basis von $\beta$-Hydroxialkylester gemäß EP 00 12 463, Beispiel II.

B8 Vernetzer auf Basis eines blockierten Isocyanats gemäß DE-AS 20 57 795, Versuch 6.

Tabelle I

| Kombination | Einbrenntemperatur °C | | | | | | | | | | |
|---|---|---|---|---|---|---|---|---|---|---|---|
| | 90 | 100 | 110 | 120 | 130 | 140 | 150 | 160 | 170 | 180 | 190 |
| A1/B1 | - | - | - | - | 4 | 2 | 1 | - | - | - | - |
| A1/B4 | - | - | - | 4 | 3 | 1-2 | 1 | - | - | - | - |
| A1/B6 | - | - | 4 | 4 | 3 | 2 | 1-2 | 1 | - | - | - |
| A2/B1 | - | - | 4 | 3 | 1 | - | - | - | - | - | - |
| A2/B2 | - | 4 | 3 | 1 | - | - | - | - | - | - | - |
| A2/B3 | - | 4 | 3 | 1 | - | - | - | - | - | - | - |
| A2/B4 | - | - | 4 | 2-3 | 1 | - | - | - | - | - | - |
| A2/B5 | - | - | - | - | - | - | - | 4 | 3 | 1-2 | 1-2 |
| A2/B6 | - | - | - | - | - | - | - | - | 4 | 3 | 1-2 |
| A2/B7 | - | - | - | - | - | - | 4 | 2 | 1 | - | - |
| A2/B8 | - | - | - | - | - | - | 4 | 3-4 | 1-2 | 1 | - |
| A3/B2 | 4 | 2-3 | 1 | - | - | - | - | - | - | - | - |
| A3/B3 | 4 | 2-3 | 1-2 | 1 | - | - | - | - | - | - | - |
| A3/B5 | 4 | 2-3 | 1-2 | 1 | - | - | - | - | - | - | - |
| A3/B6 | 4 | 4 | 2-3 | 1-2 | 1 | - | - | - | - | - | - |
| A3/B7 | - | - | - | - | 4 | 3-4 | 2 | 1 | - | - | - |

Bewertung:

1 = kein Angriff auf Oberfläche

2 = geringe Quellung, leichte Aufrauhung der Oberfläche

3 = starke Aufrauung der Oberfläche

4 = Durchgerieben bis auf Untergrund

Anwendungsbeispiel 2

Eine Lackformulierung bestehend aus

70 Teilen, bezogen auf fest, eines handelsüblichen Alkydharzes (Alkydal R40) als Lackbindemittel A1 und

1) 30 Teilen eines handelsüblichen isobutanolischen Harnstoff-Formaldehyd-Lackharzes (Plastopal FIB = Vernetzer B6) oder

2) 30 Teilen, bezogen auf fest, eines erfindungsgemäßen Harzes nach Beispiel 1 (Vernetzer B1) und

50 Teilen eines $TiO_2$-Weißpigmentes ($TiO_2$ RH 57)

werden mit einer Mischung aus 80 Teilen Xylol und 20 Teilen Glykolacetat auf einen Festgehalt von 50 % eingestellt und mit einem 100 µm-Rakel auf ein entfettetes Stahlblech aufgezogen. Der Lackfilm wird 30 Minuten lang bei 120 und 140 °C eingebrannt und anschließend den Prüfungen gemäß Tabelle II unterzogen.

Tabelle II

| | Kombination | | | |
|---|---|---|---|---|
| | A1/B1 | | A1/B6 | |
| | 120°C | 140°C | 120°C | 140°C |
| Erichsentiefung (mm) | 9,2 | 8,8 | 9,0 | 8,5 |
| Pendelhärte n. König (sec) | 105 | 136 | 90 | 120 |
| Glanz % Rem. /60°C | 94 | 93 | 90 | 87 |
| Beständigkeit gegen | | | | |
| Xylol, Note | 1-2 | 1-2 | 2 | 1-2 |
| Aceton, Note | 2 | 1-2 | 2 | 1-2 |

Bewertung:

1 = absolut beständig

2 = leichte Quellung

3 = starke Quellung

4 = unbeständig

Anwendungsbeispiel 3

Eine Mischung aus 97 Teilen (bezogen auf fest) eines Tauchlackbindemittels (Komponente A2) und 41 Teile (bezogen auf fest) eines erfindungsgemäßen Kondensationsproduktes gemäß Beispiel 9 (Komponente B4) wird durch Zugabe von 3,4 Teilen Essigsäure wasserverdünnbar und mit vollentsalztem Wasser zu einer 12 %igen wäßrigen Dispersion verarbeitet (1150 Teile). Dazu werden 137 Teile einer 50 %igen wäßrigen Dispersion gegeben, die 48 Teile eines aus Ruß und Talkum bestehenden Graupigments erhält.

Man rührt bei Raumtemperatur bis 30 °C während 48 Stunden. An kathodisch geschalteten, zinkphosphatierten Stahlprüfblechen werden innerhalb von 2 Minuten bei 250 V Lackfilme abgeschieden und 20 Minuten bei 110, 120, 130, 140 und 160 °C eingebrannt und die entstandenen glänzenden, mechanisch widerstandsfähigen Lackfilme durch 50-maliges Hin- und Herreiben mit einem mit Aceton getränkten Wattebausch auf Lösungsmittelbeständigkeit geprüft.

| Ergebnis | 110°C | 120°C | 130°C | 140°C | 160°C |
|---|---|---|---|---|---|
| Kombination A2/B4 | 4 | 1-2 | 1 | 1 | 1 |

**Bewertung:**
1 = unangreifbar
2 = leichte Aufrauung der Oberfläche
3 = starke Aufrauung der Oberfläche
4 = Abrieb bis auf Untergrund.

Beispiel 12

Das gemäß Beispiel 7 erhaltene Kondensationsproudkt wird von i-Butanol befreit, in 100 Teilen Chloroform gelöst, und bei 20 bis 40 °C 208 Teile Isophorondiisocyanat (= IPDI) portionsweise zugegeben. Nach beendeter Zugabe wird 1 Stunde am Rückfluß erhitzt, Chloroform abdestilliert, der harzartige Rückstand in 1200 Teilen i-Butanol gelöst und die organische Phase mit Wasser gewaschen.

Nach Aufkonzentrieren wurden 1488 Teile einer 60,5 %igen i-butanolischen Harzlösung erhalten.

Beispiel 13

Das gemäß Beispiel 8 erhaltene Kondensationsprodukt wird von i-Butanol befreit, und das hellgelbe harzartige Produkt in Chloroform aufgenommen.

Die so gewonnene Harzlösung wird portionsweise mit 87 Teilen Toluylendiisocyanat (= TDI) versetzt, wobei die Reaktionsmischung bei 20 bis 40 °C gehalten wird. Nach beendeter Zugabe von TDI wird 2 Stunden bei Rückflußtemperatur gerührt, anschließend Chloroform abdestilliert, der Rückstand in i-Butanol aufgenommen und auf einen Festgehalt von ca. 65 % aufkonzentriert. Ausbeute 845 Teile einer i-butanolischen hellgelben Harzlösung.

Anwendungsbeispiel 4

Eine Lackformulierung wurde hergestellt aus
70 Teilen (bezogen auf fest) eines der folgenden Lackbindemittel A1 oder A2
und
30 Teilen (bezogen auf fest) eines der erfindungsgemäßen Umsetzungsprodukte B9 oder B10 gemäß den Beispielen 12 und 13 bzw. zum Vergleich handelsüblichen Produkten B3 bis B6.

Die Mischung der beiden Komponenten wurde mit Ethanol auf ca. 50 bis 60 % eingestellt und auf ein Stahlblech mit einem 100 µm Rakel aufgezogen.

Anschließend wurden die Lacke 20 Minuten bei den in der Tabelle angegebenen Temperaturen eingebrannt. Die Prüfung der Lackfilme erfolgte durch 100 maliges Reiben mit einem mit Aceton getränkten Wattebauschs, die Ergebnisse sind in Tabelle III wiedergegeben.

A1 Handelsübliches Lackbindemittel auf Polyesterbasis (Alkydal® R40)
A2 Nicht selbstvernetzendes, kathodisch abscheidbares Bindemittel, wie in Anwendungsbeispiel 1 beschrieben.
B9 Erfindungsgemäßes Umsetzungsprodukt gemäß Beispiel 12.
B10 Erfindungsgemäßes Umsetzungsprodukt gemäß Beispiel 13.

Zum Vergleich

B5 Handelsübliches Melamin-Formaldheyd-Lackharz, butanolverethert (z.B. Luwipal®015).
B6 Handelsübliches Harnstoff-Formaldehyd-Lackharz, isobutanolverethert (z.B. Plastopal® FIB).
B7 Vernetzer auf Basis von β-Hydroxialkylester gemäß EP 00 12 463, Beispiel II.
B8 Vernetzer auf Basis eines blockierten Isocyanats gemäß DE-AS 20 57 795, Versuch 6.

Tabelle III

| Kombination | Einbrenntemperatur °C | | | | | | | | | | |
|---|---|---|---|---|---|---|---|---|---|---|---|
| | 90 | 100 | 110 | 120 | 130 | 140 | 150 | 160 | 170 | 180 | 190 |
| A1/B9 | - | - | - | - | 4 | 2 | 1 | - | - | - | - |
| A1/B10 | - | - | - | - | 4 | 1-2 | 1 | - | - | - | - |
| A1/B6 | - | - | - | - | 4 | 2-3 | 1-2 | 1 | - | - | - |
| A2/B9 | - | - | 4 | 3 | 1 | - | - | - | - | - | - |
| A2/B10 | - | - | 4 | 2 | 1 | - | - | - | - | - | - |
| A2/B5 | - | - | - | - | - | - | - | 4 | 3 | 1-2 | 1-2 |
| A2/B6 | - | - | - | - | - | - | - | - | 4 | 3 | 1-2 |
| A2/B7 | - | - | - | - | - | - | - | 4 | 2 | 1 | - |
| A2/B8 | - | - | - | - | - | - | 4 | 3-4 | 1-2 | 1 | - |

Bewertung:
1 = kein Angriff auf Oberfläche
2 = geringe Quellung, leichte Aufrauhung der Oberfläche
3 = starke Aufrauhung der Oberfläche
4 = Durchgerieben bis auf Untergrund

Anwendungsbeispiel 5

Eine Lackformulierung bestehend aus
70 Teilen, bezogen auf fest, eines handelsüblichen Alkydharzes (Alkydal R40) als Lackbindemittel A1 und
1) 30 Teilen eines handelsüblichen isobutanolischen Harnstoff-Formaldehyd-Lackharzes (Plastopal FIB = Vernetzer B6) oder
2) 30 Teilen, bezogen auf fest, eines erfindungsgemäßen Harzes nach Beispiel 12 (Vernetzer B9) und
50 Teilen eines $TiO_2$-Weißpigmentes ($TiO_2$ RH 57)
wurden mit einer Mischung aus 80 Teilen Xylol und 20 Teilen Glykolacetat auf einen Festgehalt von 50 % eingestellt und mit einem 100 µm-Rakel auf ein entfettetes Stahlblech aufgezogen. Der Lackfilm wurde 30 Minuten lang bei 120 und 140°C eingebrannt und anschließend den Prüfungen gemäß Tabelle IV unterzogen.

Tabelle IV

| | Kombination | | | |
|---|---|---|---|---|
| | A1/B9 | | A1/B6 | |
| | 120°C | 140°C | 120°C | 140°C |
| Erichsentiefung (mm) | 9,4 | 8,6 | 9,2 | 8,5 |
| Pendelhärte n. König (sec) | 110 | 136 | 90 | 120 |
| Glanz % Rem. /60°C | 95 | 92 | 90 | 87 |
| Beständigkeit gegen | | | | |
| Xylol, Note | 1-2 | 1-2 | 2 | 1-2 |
| Aceton, Note | 2 | 1-2 | 2 | 1-2 |

Bewertung:
1 = absolut beständig
2 = leichte Quellung
3 = starke Quellung
4 = unbeständig

Anwendungsbeispiel 6

Eine Mischung aus 97 Teilen (bezogen auf fest) eines Tauchlackbindemittels (Komponente A2) und 41 Teile (bezogen auf fest) eines erfindungsgemäßen Umsetzungsproduktes gemäß Beispiel 12 (Komponente B9) wird durch Zugabe von 3,4 Teilen Essigsäure wasserverdünnbar und mit vollentsalztem Wasser zu einer 12 %igen wäßrigen Dispersion verarbeitet (1150 Teile).

Dazu werden 137 Teile einer 50 %igen wäßrigen Dispersion gegeben, die 48 Teile eines aus Ruß und Talkum bestehenden Graupigments erhält.

Man rührt bei Raumtemperatur bis 30 °C während 48 Stunden. An kathodisch geschalteten, zinkphosphatierten Stahlprüfblechen werden innerhalb von 2 Minuten bei 250 V Lackfilme abgeschieden und 20 Minuten bei 110, 120, 130, 140 und 160 °C eingebrannt und die entstandenen glänzenden, mechanisch widerstandsfähigen Lackfilme durch 50-maliges Hin- und Herreiben mit einem mit Aceton getränkten Wattebausch auf Lösungsmittelbeständigkeit geprüft.

| Ergebnis | 110°C | 120°C | 130°C | 140°C | 160°C |
|---|---|---|---|---|---|
| Kombination A2/B9 | 4 | 3 | 1-2 | 1 | 1 |

Bewertung:
1 = unangreifbar
2 = leichte Aufrauhung der Oberfläche
3 = starke Aufrauhung der Oberfläche
4 = Abrieb bis auf Untergrund.

Beispiel 14

α) Herstellung eines Kondensationsproduktes, das im wesentlichen Strukturen der Formel

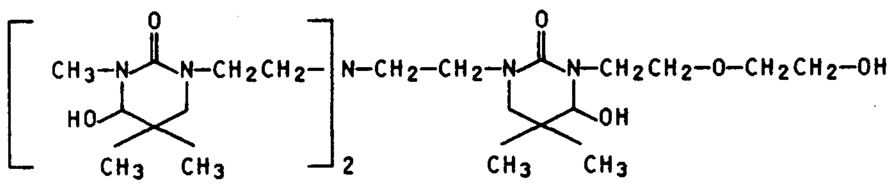

enthält:
Wie in Beispiel 8 beschrieben, werden 176 Teile N,N'-Dimethylharnstoff, 60 Teile Harnstoff und 146 Teile Tris-2-aminoethyl-amin innerhalb von 3,5 Stunden von 120 auf 160°C erhitzt, anschließend 105 Teile 2,2-Aminoethoxiethanol zugesetzt und weitere 3 Stunden bei Temperaturen von 140 bis 160°C ansteigend erhitzt. Dann werden 200 Teile Wasser und 50 Teile Eisessig zugesetzt. Nach Zugabe von 225 Teilen 40 %iger wäßriger Formaldehydlösung und 216 Teilen Isobutyraldehyd wird auf Rückflußtemperatur erwärmt und innerhalb von 20 Minuten 150 Teile conc. Salpetersäure zugegeben. Die Rückflußtemperatur steigt dabei auf ca. 100°C an. Bei dieser Temperatur wird zwei Stunden lang gerührt, abgekühlt und mit Natronlauge neutralisiert. Zur Entfernung des Salzes wird das Reaktionsgemisch aufkonzentriert, der Rückstand in Methanol aufgenommen, filtriert und erneut aufkonzentriert.
β) Herstellung des erfindungsgemäßen Umsetzungsproduktes: Das unter α) erhaltene Konzentrat wird in 1000 Teilen Eisessig aufgenommen und 220 Teile 1,2,4,5-Benzoltetracarbonsäureanhydrid zugegeben. Die Mischung wird 3 Stunden bei 80°C gerührt, anschließend Essigsäure abdestilliert und der Rückstand in i-Butanol aufgenommen. Die organische Harzlösung wird mit Wasser und Natronlauge säure- und salzfrei gewaschen und anschließend auf 847 Teile einer 62 %igen i-butanolischen Harzlösung aufkonzentriert.

Anwendungsbeispiel 7

Eine Lackformulierung wurde hergestellt aus
70 Teilen (bezogen auf fest) eines der folgenden Lackbindemittel A1 bis A3
und
30 Teilen (bezogen auf fest) des erfindungsgemäßen Umsetzungsproduktes B11 gemäß Beispiel 14 bzw. zum Vergleich handelsüblichen Produkten B5 bis B8.

Die Mischung der beiden Komponenten wurde mit Ethanol auf ca. 50 bis 60 % eingestellt und auf ein Stahlblech mit einem 100 µm Rakel aufgezogen.

Anschließend werden die Lacke 20 Minuten bei den in der Tabelle angegebenen Temperaturen eingebrannt. Die Prüfung der Lackfilme erfolgte durch 100 maliges Reiben mit einem mit Aceton getränkten Wattebauschs, die Ergebnisse sind in Tabelle V wiedergegeben.

A1 = Handelsübliches Lackbindemittel auf Polyesterbasis (Alkydal® R40)
A2 = Nicht selbstvernetzendes, kathodisch abscheidbares Bindemittel, wie in Anwendungsbeispiel 1 beschrieben.
A3 = OH-gruppenhaltiges Polyacrylat, wie in Anwendungsbeispiel 1 beschrieben.
B11 = Erfindungsgemäßes Kondensationsprodukt gemäß Beispiel 14.

Zum Vergleich

B5 = Handelsübliches Melamin-Formaldheyd-Lackharz, butanolverethert (z.B. Luwipal®015).
B6 = Handelsübliches Harnstoff-Formaldehyd-Lackharz, isobutanolverethert (z.B. Plastopal® FIB).
B7 = Vernetzer auf Basis von β-Hydroxialkylester gemäß EP 00 12 463, Beispiel II.
B8 = Vernetzer auf Basis eines blockierten Isocyanats gemäß DE-AS 20 57 795, Versuch 6.

Tabelle V

| Kombination | Einbrenntemperatur °C | | | | | | | | | | |
|---|---|---|---|---|---|---|---|---|---|---|---|
| | 90 | 100 | 110 | 120 | 130 | 140 | 150 | 160 | 170 | 180 | 190 |
| A1/B11 | - | - | - | - | 4 | 2 | 1 | - | - | - | - |
| A2/B11 | - | - | 4 | 3 | 1 | - | - | - | - | - | - |
| A2/B5 | - | - | - | - | - | - | - | 4 | 3 | 1-2 | 1-2 |
| A2/B6 | - | - | - | - | - | - | - | - | 4 | 3 | 1-2 |
| A2/B7 | - | - | - | - | - | - | 4 | 2 | 1 | - | - |
| A2/B8 | - | - | - | - | - | - | 4 | 3-4 | 1-2 | 1 | - |
| A3/B11 | 4 | 2-3 | 1 | - | - | - | - | - | - | - | - |
| A3/B5 | 4 | 3 | 1-2 | 1 | - | - | - | - | - | - | - |
| A3/B6 | 4 | 3 | 1-2 | 1 | - | - | - | - | - | - | - |
| A3/B8 | - | - | - | - | 4 | 3-4 | 2 | 1 | - | - | - |

Bewertung:

1 = kein Angriff auf Oberfläche

2 = geringe Quellung, leichte Aufrauhung der Oberfläche

3 = starke Aufrauhung der Oberfläche

4 = Durchgerieben bis auf Untergrund

Anwendungsbeispiel 8

Eine Lackformulierung bestehend aus
70 Teilen, bezogen auf fest, eines handelsüblichen Alkydharzes (Alkydal R40) als Lackbindemittel A1 und
1) 30 Teilen eines handelsüblichen isobutanolischen Harnstoff-Formaldehyd-Lackharzes (Plastopal FIB = Vernetzer B6) oder
2) 30 Teilen, bezogen auf fest, eines erfindungsgemäßen Harzes nach Beispiel 14 (Vernetzer B11) und
50 Teilen eines $TiO_2$-Weißpigmentes ($TiO_2$ RH 57)
werden mit einer Mischung aus 80 Teilen Xylol und 20 Teilen Glykolacetat auf einen Festgehalt von 50 % eingestellt und mit einem 100 µm-Rakel auf ein entfettetes Stahlblech aufgezogen. Der Lackfilm wird 30 Minuten lang bei 120 und 140°C eingebrannt und anschließend den Prüfungen gemäß Tabelle VI unterzogen.

Tabelle VI

| | Kombination | | | |
|---|---|---|---|---|
| | A1/B11 | | A1/B6 | |
| | 120°C | 140°C | 120°C | 140°C |
| Erichsentiefung (mm) | 9,4 | 8,6 | 9,0 | 8,5 |
| Pendelhärte n. König (sec) | 108 | 146 | 90 | 122 |
| Glanz % Rem. /60°C | 94 | 90 | 90 | 87 |
| Beständigkeit gegen | | | | |
| Xylol, Note | 1-2 | 1-2 | 2 | 1-2 |
| Aceton, Note | 1-2 | 1-2 | 2 | 1-2 |

Bewertung:
1 = absolut beständig
2 = leichte Quellung
3 = starke Quellung
4 = unbeständig

Beispiel 15

α) Herstellung eines Kondensationproduktes, das im wesentlichen Strukturen der Formel

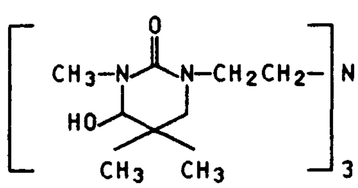

enthält:
Wie in Beispiel 5 beschrieben, werden 292 Teile Tris-2-aminoethylamin und 528 Teile N,N'-Dimethylharnstoff je 1 Stunde bei 120 und 140 °C, dann 2 Stunden bei 150 °C und abschließend 0,5 Stunden bei 160 °C unter Stickstoff gerührt. Die Viskosität der Reaktionsmischung steigt gegen Ende der Reaktion deutlich an. Anschließend werden 350 Teile Wasser und 50 Teile Eisessig zugegeben und unter Stickstoff 1 Stunde am Rückfluß gerührt. Anschließend werden 450 Teile Formaldehyd (40 %ig in Wasser) und 144 Teile conc. Salpetersäure zugesetzt und auf 65 °C erhitzt. Dann werden 432 Teile Isobutyraldehyd so rasch zugetropft, daß die Reaktionsmischung am lebhaften Rückfluß bleibt. Die Rückflußtemperatur steigt auf ca. 103 °C an, und die Mischung wird 2 Stunden bei dieser Temperatur gerührt. Nach Abkühlen wird mit Natronlauge pH 3,5 eingestellt und 800 Teile Butanol zugegeben.
β) Verknüpfung mit Bisphenol-A (= Herstellung des erfindungsgemäßen Umsetzungsproduktes):
Nach Zugabe von 228 Teilen Bisphenol-A wird bei Normaldruck während 3 Stunden bei ca. 100 °C Wasser ausgekreist, abgekühlt und mit Natronlauge pH 6,5 eingestellt. Nach Abtrennen der wäßrigen Phase, Waschen der organischen Phase mit Wasser und Aufkonzentrieren im Vakuum werden 1538 Teile einer 65 %igen Harzlösung erhalten.

Beispiel 16

α) Herstellung eines Kondensationsproduktes, das im wesentlichen Strukturen der Formel

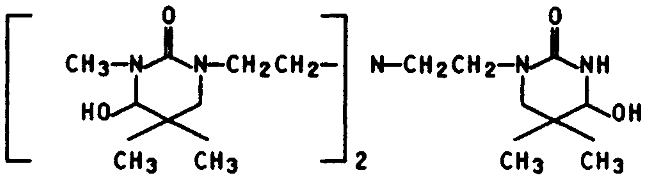

enthält:
352 Teile N,N'-Dimethylharnstoff, 120 Teile Harnstoff und 292 Teile Tris-2-aminoethyl-amin werden in 45 Minuten auf 120 °C erhitzt und innerhalb von 3,5 Stunden kontinuierlich auf 160 °C aufgeheizt, wobei Stickstoff in die Schmelze geleitet wird.

Nach Zugabe von 400 Teilen Wasser und 100 Teilen Eisessig wird auf 50 °C abgekühlt, 450 Teile einer 40 %igen wäßrigen Formaldehydlösung und 432 Teile Isobutyraldehyd zugegeben und auf Rückflußtemperatur erhitzt. Nach portionsweiser Zugabe von 240 Teilen konzentrierter Salpetersäure steigt die Temperatur auf 100 °C an. Bei dieser Temperatur wird 2 Stunden gerührt, abgekühlt, mit Natronlauge pH 3,8 eingestellt und 2000 Teile i-Butanol zugegeben.

β) Verknüpfung mit der Dimethylolverbindung von Bisphenol-A ( = Herstellung des erfindungsgemäßen Umsetzungsproduktes):
1000 Teile einer i-butanolischen Lösung des Methylolierungsproduktes von 1 Mol Bisphenol-A mit 2 Mol Formaldehyd werden der unter α) erzeugten Harzlösung zugesetzt und 3,5 Stunden Wasser ausgekreist. Nach Abkühlen wird ein pH-Wert von 7,0 eingestellt, die Harzlösung zweimal mit je 1000 Teilen Wasser gewaschen und die organische Phase auf 1780 Teile einer i-butanolischen Harzlösung mit Festharzanteil von 68 % aufkonzentriert.

Anwendungsbeispiel 9

Eine Lackformulierung wurde hergestellt aus
70 Teilen (bezogen auf fest) eines der folgenden Lackbindemittel A1 bis A3
und
30 Teilen (bezogen auf fest) eines der erfindungsgemäßen Umsetzungsprodukte B12 oder B13 gemäß den Beispielen 15 und 16 bzw. zum Vergleich handelsüblichen Produkten B5 bis B8.

Die Mischung der beiden Komponenten wurde mit Ethanol auf ca. 50 bis 60 % eingestellt und auf ein Stahlblech mit einem 100 µm Rakel aufgezogen.

Anschließend wurden die Lacke 20 Minuten bei den in der Tabelle angegebenen Temperaturen engebrannt. Die Prüfung der Lackfilme erfolgte durch 100 maliges Reiben mit einem mit Aceton getränkten Wattebauschs, die Ergebnisse sind in Tabelle VII wiedergegeben.

A1 = Handelsübliches Lackbindemittel auf Polyesterbasis (Alkydal® R40)
A2 = Nicht selbstvernetzendes, kathodisch abscheidbares Bindemittel, wie in Anwendungsbeispiel 1 beschrieben.
A3 = OH-gruppenhaltiges Polyacrylat, wie in Anwendungsbeispiel 1 beschrieben.
B12 = Erfindungsgemäßes Umsetzungsprodukt gemäß Beispiel 15.
B13 = Erfindungsgemäßes Umsetzungsprodukt gemäß Beispiel 16.

Zum Vergleich

B5 = Handelsübliches Melamin-Formaldheyd-Lackharz, butanolverehert (z.B. Luwipal®015
B6 = Handelsübliches Harnstoff-Formaldehyd-Lackharz, isobutanolverethert (z.B. Plastopal® FIB).
B7 = Vernetzer auf Basis von β-Hydroxialkylester gemäß EP 00 12 463, Beispiel II.
B8 = Vernetzer auf Basis eines blockierten Isocyanats gemäß DE-AS 20 57 795, Versuch 6.

Tabelle VII

| Kombination | Einbrenntemperatur °C | | | | | | | | | | |
|---|---|---|---|---|---|---|---|---|---|---|---|
| | 90 | 100 | 110 | 120 | 130 | 140 | 150 | 160 | 170 | 180 | 190 |
| A1/B12 | - | - | - | - | 4 | 2 | 1 | - | - | - | - |
| A1/B13 | - | - | - | 4 | 3 | 1-2 | 1 | - | - | - | - |
| A1/B6 | - | - | - | 4 | 3-4 | 2-3 | 1-2 | - | - | - | - |
| A2/B12 | - | - | 4 | 3 | 1 | - | - | - | - | - | - |
| A2/B13 | - | - | 4 | 2-3 | 1 | - | - | - | - | - | - |
| A2/B5 | - | - | - | - | - | - | - | 4 | 3 | 1-2 | 1-2 |
| A2/B6 | - | - | - | - | - | - | - | - | 4 | 3 | 1-2 |
| A2/B7 | - | - | - | - | - | - | 4 | 2 | 1 | - | - |
| A2/B8 | - | - | - | - | - | - | 4 | 3-4 | 1-2 | 1 | - |
| A3/B12 | 4 | 2-3 | 1 | - | - | - | - | - | - | - | - |
| A3/B13 | 4 | 2-3 | 1-2 | 1 | - | - | - | - | - | - | - |
| A3/B5 | 4 | 2-3 | 2 | 1-2 | 1 | - | - | - | - | - | - |
| A3/B6 | 4 | 4 | 2-3 | 1-2 | 1 | - | - | - | - | - | - |
| A3/B8 | - | - | - | - | - | - | 4 | 4 | 2-3 | 1 | - |

Bewertung:

1 = kein Angriff auf Oberfläche

2 = geringe Quellung, leichte Aufrauhung der Oberfläche

3 = starke Aufrauhung der Oberfläche

4 = Durchgerieben bis auf Untergrund

Anwendungsbeispiel 10

Eine Lackformulierung bestehend aus

70 Teilen, bezogen auf fest, eines handelsüblichen Alkydharzes (Alkydal R40) als Lackbindemittel A1 und

1) 30 Teilen eines handelsüblichen isobutanolischen Harnstoff-Formaldehyd-Lackharzes (Plastopal FIB = Vernetzer B) oder

2) 30 Teilen, bezogen auf fest, eines erfindungsgemäßen Harzes nach Beispiel 15 (Vernetzer B12) und

50 Teilen eines $TiO_2$-Weißpigmentes ($TiO_2$ RH 57)

wurden mit einer Mischung aus 80 Teilen Xylol und 20 Teilen Glykolacetat auf einen Festgehalt von 50 % eingestellt und mit einem 100 µm-Rakel auf ein entfettetes Stahlblech aufgezogen. Der Lackfilm wird 30 Minuten lang bei 120 und 140 °C eingebrannt und anschließend den Prüfungen gemäß Tabelle VIII unterzogen.

Tabelle VIII

| | Kombination | | | |
|---|---|---|---|---|
| | A1/B12 | | A1/B6 | |
| | 120°C | 140°C | 120°C | 140°C |
| Erichsentiefung (mm) | 9.0 | 8.7 | 9,0 | 8,5 |
| Pendelhärte n. König (sec) | 105 | 144 | 90 | 120 |
| Glanz % Rem. /60°C | 94 | 93 | 90 | 87 |
| Beständigkeit gegen | | | | |
| Xylol, Note | 1-2 | 1-2 | 2 | 1-2 |
| Aceton, Note | 1-2 | 1-2 | 2 | 1-2 |

Bewertung:
1 = absolut beständig
2 = leichte Quellung
3 = starke Quellung
4 = unbeständig

Anwendungsbeispiel 11

Eine Mischung aus 97 Teilen (bezogen auf fest) eines Tauchlackbindemittels (Komponente A2) und 41 Teile (bezogen auf fest) eines erfindungsgemäßen Kondensationsproduktes gemäß Beispiel 15 (Komponente B12) wurde durch Zugabe von 3, 4 Teilen Essigsäure wasserverdünnbar und mit vollentsalztem Wasser zu einer 12 %igen wäßrigen Dispersion verarbeitet (1150 Teile). Dazu wurden 137 Teile einer 50 %igen wäßrigen Dispersion gegeben, die 48 Teile eines aus Ruß und Talkum bestehenden Graupigments enthielt.

Man rührte bei Raumtemperatur bis 30 °C während 48 Stunden. An kathodisch geschalteten, zinkphosphatierten Stahlprüfblechen wurden innerhalb von 2 minuten bei 250 V Lackfilme abgeschieden und 20 Minuten bei 110, 120, 130, 140 und 160 °C eingebrannt und die entstandenen glänzenden, mechanisch widerstandsfähigen Lackfilme durch 50-maliges Hin- und Herreiben mit einem mit Aceton getränkten Wattebausch auf Lösungsmittelbeständigkeit geprüft.

| Ergebnis | 110°C | 120°C | 130°C | 140°C | 160°C |
|---|---|---|---|---|---|
| Kombination A2/B12 | 4 | 2-3 | 1-2 | 1 | 1 |

Bewertung:
1 = unangreifbar
2 = leichte Aufrauhung der Oberfläche
3 = starke Aufrauhung der Oberfläche
4 = Abrieb bis auf Untergrund.

**Patentansprüche**

**Patentansprüche für folgende Vertragsstaaten : DE, FR, GB, IT, NL, SE**

1. Umsetzetzungsprodukte von Kondensationsprodukten, die im wesentlichen substituierte Propylenharnstoffe der allgemeinen Formeln (I) und/oder (II)

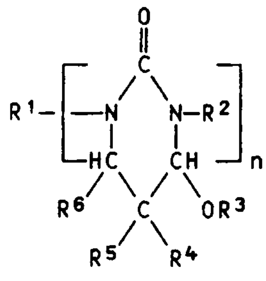

(I)

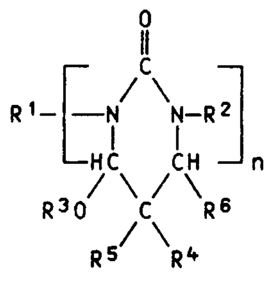

(II)

enthalten, worin $R^1$ einen mindestens zweiwertigen organischen, 4 bis 60 kohlenstoffatome enthaltenden geradkettigen oder verzweigten Alkylen-, Cycloakylen-, Oxaalkylen- oder Azaalkylenrest bedeutet, wobei der Rest $R^1$ gegebenenfalls eine oder mehrere Hydroxylgruppen und/oder mit Hydroxyalkyl-, Hydroxycycloalkyl-, Hydroxyoxaalkyl- und/oder Hydroxyazaalkylgruppen substituierte Harnstoff-, Carbamat-, Carbonamid- oder Sulfonamidgruppen enthalten kann,
$R^2$, $R^3$, $R^4$, $R^5$ und $R^6$ untereinander gleich oder verschieden sind und für Wasserstoff, Alkylreste mit 1 bis 18 Kohlenstoffatomen, Cycloalkylreste mit 5 bis 15 Kohlenstoffatomen, Arylreste mit 6 bis 15 Kohlenstoffatomen, Aralkylreste mit 7 bis 16 Kohlenstoffatomen, Oxaalkyl- oder Azaalkylreste mit 2 bis 18 Kohlenstoffatomen, stehen, und $R^2$ zusätzlich für einen Hydroxyalkylrest stehen kann, mit der Maßgabe, daß für den Fall, daß $R^2$ für einen Hydroxyalkylrest steht, dieser 4 bis 18 Kohlenstoffatome enthält, und $R^2$ nicht für einen Aryl- oder Aralkylrest steht,
und $n = 2$ bis 20 ist, wobei die Wertigkeit von $R^1$ n entspricht und die enzelnen Reste $R^2$ bis $R^6$ der n Propylenharnstoffeinheiten der Formeln (I) und/oder (II) innerhalb der oben angegebenen Definitionen untereinander jeweils gleich oder verschieden sein können, mit
Di- und/oder Polyisocyanaten,
Derivaten von Di- und/oder Polycarbonsäuren oder
p-substituierten Phenolen und/oder deren o-Methylolderivaten, mit der Maßgabe, daß bei Verwendung von o-Methylolphenolen mindestens einer der Reste $R^1$ oder $R^2$ der Formeln (I) und/oder (II) eine Hydroxylgruppe enthält oder der Rest $R^2$ für Wasserstoff steht.

2. Kondensationsprodukte die im wesentlichen substituierte Propylenharnstoffe der allgemeinen Formeln (I) und/oder (II)

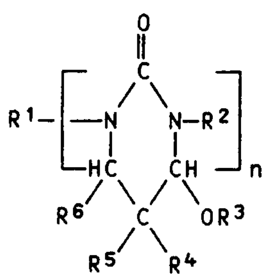

(I)

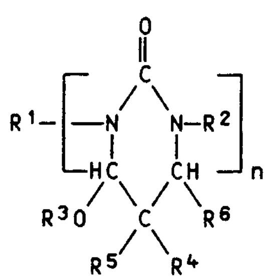

(II)

enthalten, worin $R^1$ einen mindestens zweiwertigen organischen, 4 bis 60 Kohlenstoffatome enthaltenden geradkettigen oder verzweigten Alkylen-, Cycloalkylen-, Oxaalkylen- oder Azaalkylenrest bedeutet, wobei der Rest $R^1$ gegebenenfalls eine oder mehrere Hydroxylgruppen und/oder mit Hydroxyalkyl-, Hydroxycycloalkyl-, Hydroxyoxaalkyl- und/oder Hydroxyazaalkylgruppen substituierte Harnstoff-, Carbamat-, Carbonamid- oder Sulfonamidgruppen enthalten kann,
$R^2$, $R^3$, $R^4$, $R^5$ und $R^6$ untereinander gleich oder verschieden sind und für Wasserstoff, Alkylreste mit 1

bis 18 Kohlenstoffatomen, Cycloalkylreste mit 5 bis 15 Kohlenstoffatomen, Arylreste mit 6 bis 15 Kohlenstoffatomen, Aralkylreste mit 7 bis 16 Kohlenstoffatomen, Oxaalkyl- oder Azaalkylreste mit 2 bis 18 Kohlenstoffatomen, stehen, und $R^2$ zusätzlich für einen Hydroxalkylrest stehen kann, mit der Maßgabe, daß für den Fall, daß $R^2$ für einen Hydroxyalkylrest steht, dieser 4 bis 18 Kohlenstoffatome enthält, und $R^2$ nicht für einen Aryl- oder Aralkylrest steht,
und n = 2 bis 20 ist, wobei die Wertigkeit von $R^1$ n entspricht und die einzelnen Reste $R^2$ bis $R^6$ der n Propylenharnstoffeinheiten der Formeln (I) und/oder (III) innerhalb der oben angegebenen Definitionen untereinander jeweils gleich oder verschieden sein können.

**3.** Verfahren zur Herstellung der Kondensationsprodukte nach Anspruch 2, dadurch gekennzeichnet, daß man zunächst
a) primäre Di- und/oder Polyamine der allgemeinen Formel (III)

$$R^1\!-\!\left[NH_2\right]_n$$

mit
b) Harnstoff, monosubstituierten oder symmetrisch disubstituierten Harnstoffen der allgemeinen Formel (IV) $R^2NH\text{-}CO\text{-}NHR^2$
bei Temperaturen von 100 bis 200 °C, gegebenenfalls in Anwesenheit von sauren oder basischen Katalysatoren sowie gegebenenfalls in Anwesenheit von
c) Dihydroxiverbindungen, Polyhydroxiverbindungen, Alkanolaminen, Hydroxyalkylcarbonsäuren und/oder Hydroxyalkylsulfonsäuren oder Hydroxyalkylsulfonsäureestern
umsetzt und die so erhaltenen Produkte mit
d) 1,4 bis 2,5 Mol eines oder mehrerer Aldehyde der allgemeinen Formel (V)

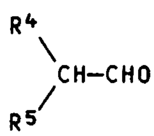 und/oder (VI) $R^6$-CHO

pro Mol Harnstoff (b) in Gegenwart saurer Katalysatoren bei Temperaturen von 50 bis 150 °C, gegebenenfalls in Anwesenheit eines Lösungsmittels umsetzt, mit der Maßgabe, daß mindestens 0,7 Mol pro Mol eingesetztem Harnstoff (b) eines Aldehyds (d) verwendet werden, der mindestens ein in $\alpha$-Stellung zur Aldehydgruppe befindliches Wasserstoffatom enthält,
wobei $R^1$ und $R^6$ und n die in Anspruch 2 angegebene Bedeutung haben.

**4.** Verfahren zur Herstellung der Kondensationsprodukte nach Anspruch 2, dadurch gekennzeichnet, daß man
a) primäre Monoamine der allgemeinen Formel (VII) $R^2$-$NH_2$ oder primäre Di- und/oder Polyamine der allgemeinen Formel (III)

$$R^1\!-\!\left[NH_2\right]_n$$

zunächst mit
e) Monoisocyanaten der allgemeinen Formel (VIII) $R^2$-NCO oder Di- und/oder Polyisocyanaten der allgemeinen Formel (IX)

$$R^1\!-\!\left[NCO\right]_n$$

umsetzt mit der Maßgabe, daß für den Fall, daß als Komponente (a) primäre Monoamine $R^2$-$NH_2$ eingesetzt werden, diese mit Di- und/oder Polyisocyanaten

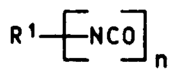

umgesetzt werden, und für den Fall, daß als Komponente (e) Monoisocyanate $R^2$-NCO eingesetzt werden, diese mit Di- und/oder Polyaminen

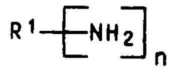

umgesetzt werden und die so erhaltenen Di- oder Polyharnstoffe mit
d) 1,4 bis 2,5 Mol eines oder mehrerer Aldehyde

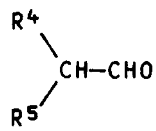 und/oder $R^6$—CHO

pro Mol der bei der Umsetzung von (a) mit (e) gebildeten Harnstoffeinheiten in Gegenwart saurer Katalysatoren bei Temperaturen von 50 bis 150 ° C, gegebenenfalls in Anwesenheit eines Lösungsmittels, umsetzt, mit der Maßgabe, daß mindestens 0,7 Mol pro Mol der durch Umsetzung von a) mit e) gebildeten Harnstoffeinheiten mindestens eines Aldehyds d) verwendet werden, der mindestens ein in α-Stellung zur Aldehydgruppe befindliches Wasserstoffatom enthält,
wobie $R^1$ bis $R^6$ und n die in Anspruch 2 angegebene Bedeutung haben.

**5.** Verfahren nach Anspruch 4,dadurch gekennzeichnet, daß als Aldehyd d) Isobutyraldehyd oder ein Gemisch von Isobutyraldehyd mit Formaldehyd verwendet wird.

**6.** Verfahren zur Herstellung der Umsetzungssprodukte nach Anspruch 1, dadurch gekennzeichnet, daß man zunächst
a) primäre Di- und/oder Polyamine der allgemeinen Formel (III)

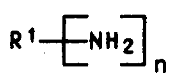

mit
b) Harnstoff, monosubstituierten oder symmetrisch disubstituierten Harnstoffen der allgemeinen Formel (IV) $R^2$NH-CO-NH$R^2$
bei Temperaturen von 100 bis 200 ° C, gegebenfalls in Anwesenheit von sauren oder basischen Katalysatoren sowie gegebenenfalls in Anwesenheit von
c) Dihydroxiverbindungen, Polyhydroxiverbindungen, Alkanolaminen, Hydroxyalkylcarbonsäuren und/oder Hydroxyalkylsulfonsäuren oder Hydroxyalkylsulfonsäureestern
umsetzt und die so erhaltenen Di- und/oder Polyharnstoffe mit
d) 1,4 bis 2,5 Mol eines oder mehrerer Aldehyde der allgemeinen Formel (V)

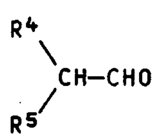 und/oder (VI) $R^6$—CHO

pro Mol Harnstoff (b) in Gegenwart saurer Katalysatoren bei Temperaturen von 50 bis 150 ° C, gegebenenfalls in Anwesenheit eines Lösungsmittels umsetzt, mit der Maßgabe, daß mindestens 0,7 Mol pro Mol eingesetztem Harnstoff (b) eines Aldehyds (d) verwendet werden, der mindestens ein in α-Stellung zur Aldehydgruppe befindliches Wasserstoffatom enthält,

wobei $R^1$ bis $R^6$ und n die in Anspruch 1 angegebene Bedeutung haben, und die so erhaltenen Produkte mit
Di- und/oder Polyisocyanaten,
Derivaten von Di- und/oder Polycarbonsäuren oder
p-substituierten Phenolen und/oder deren o-Methylolderivaten umsetzt, mit der Maßgabe, daß pro Mol eingesetzten Harnstoffs b) 0,25 bis 1,0 Mol Isocyanatgruppen des Di- und/oder Polyisocyanats, Säurehalogenid- und/oder -anhydridgruppen oder p-substituiertes Phenol und/oder dessen o-Methylolderivat verwendet werden.

**7.** Verfahren zur Herstellung der Umsetzungsprodukte nach Anspruch 1, dadurch gekennzeichnet, daß man
a) primäre Monoamine der allgemeinen Formel (VII) $R^2$-$NH_2$ oder primäre Di- und/oder Polyamine der allgemeinen Formel (III)

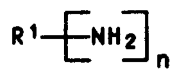

zunächst mit
e) Monoisocyanaten der allgemeinen Formel (VIII) $R^2$-NCO oder Di- und/oder Polyisocyanaten der allgemeinen Formel (IX)

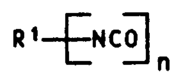

umsetzt mit der Maßgabe, daß für den Fall, daß als Komponente (a) primäre Monoamine $R^2$-$NH_2$ eingesetzt werden, diese mit Di- und/oder Polyisocyanaten

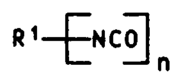

umgesetzt werden, und für den Fall, daß als Komponente (e) Monoisocyanate $R^2$-NCO eingesetzt werden, diese mit Di- und/oder Polyaminen

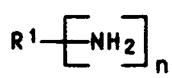

umgesetzt werden und die so erhaltenen Di- oder Polyharnstoffe mit
d) 1,4 bis 2,5 Mol eines oder mehrerer Aldehyde

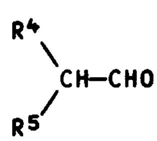 und/oder $R^6$—CHO

pro Mol der bei der Umsetzung von (a) mit (e) gebildeten Harnstoffeinheiten in Gegenwart saurer Katalysatoren bei Temperaturen von 50 bis 150 ° C, gegebenenfalls in Anwesenheit eines Lösungsmittels, umsetzt, mit der Maßgabe, daß mindestens 0,7 Mol pro Mol der durch Umsetzung von a) mit e) gebildeten Harnstoffeinheiten mindestens eines Aldehyds d) verwendet werden, der mindestens ein in $\alpha$-Stellung zur Aldehydgruppe befindliches Wasserstoffatom enthält, wobei $R^1$ bis $R^6$ und n die in Anspruch 1 angegebene Bedeutung haben, und die so erhaltenen Produkte mit
Di- und/oder Polyisocyanaten,
Derivaten von Di- und/oder Polycarbonsäuren oder
p-substituierten Phenolen und/oder deren o-Methylolderivaten umsetzt, mit der Maßgabe, daß pro

Mol eingesetzten Harnstoffs b) 0,25 bis 1,0 Mol Isocyanatgruppen des Di- und/oder Polyisocyanats, Säurehalogenid und/oder -anhydridgruppen oder p-substituiertes Phenol und/oder dessen o-Methylolderivat verwendet werden.

**8.** Verfahren nach Anspruch 7, dadurch gekennzeichnet, daß als Aldehyd d) Isobutyraldehyd oder ein Gemisch von Isobutyraldehyd mit Formaldehyd verwendet wird.

**9.** Hitzehärtbares überzugsmittel, dadurch gekennzeichnet, daß es als Bindemittel ein Gemisch aus
(A) mindestens einem Polyadditions-, Polykondensations- oder Polymerisationsprodukt mit einem mittleren Molekulargewicht $\overline{M}_n$ von 500 bis 10 000, welches im Mittel pro Molekül mindestens zwei Hydroxylgruppen sowie zusätzlich gegebenenfalls primäre und/oder sekundäre und/oder tertiäre Aminogruppen besitzt und
(B) einem Umsetzungsprodukt nach Anspruch 1
enthält.

**10.** Hitzehärtbares Überzugsmittel, dadurch gekennzeichnet, daß es als Bindemittel ein Gemisch aus
(A) mindestens einem Polyadditions-, Polykondensations- oder Polymerisationsprodukt mit einem mittleren Molekulargewicht $\overline{M}_n$ von 500 bis 10 000, welches im Mittel pro Molekül mindestens zwei Hydroxylgruppen sowie zusätzlich gegebenenfalls primäre und/oder sekundäre und/oder tertiäre Aminogruppen besitzt und
(B) einem Kondensationsprodukt nach Anspruch 2
enthält.

**11.** Überzugsmittel nach Anspruch 9 oder 10, dadurch gekennzeichnet, daß die Komponenente (A) ein Polyesterharz ist.

**12.** Überzugsmittel nach Anspruch 9 oder 10, dadurch gekennzeichnet, daß die Komponente (A) eine Umsetzungsprodukt eines Epoxidharzes mit mindestens einem Amin, Alkohol oder Mercaptan ist.

**13.** Überzugsmittel nach Anspruch 9 oder 10, dadurch gekennzeichnet, daß die Komponente (A) ein hydroxylgruppenhaltiges Polyacrylatharz ist.

**14.** Verwendung der Überzugsmittel nach einem der Ansprüche 9 bis 13 für lösungsmittelhaltige Einbrennlacke.

**15.** Überzugsmittel nach einem der Ansprüche 9 bis 13, dadurch gekennzeichnet, daß sie nach zumindest teilweiser Neutralisation mit einer Säure wasserverdünnbar sind.

**16.** Verwendung der Überzugsmitel nach Anspruch 15 zur Herstellung Kathodisch abscheidbarer Elektrotauchlacke.

**17.** Verwendung der Überzugsmittel nach Anspruch 15 für wäßrige Einbrennlacke.

**18.** Wäßriges Lackbad für die kathodische Elektrotauchlackierung, dadurch gekennzeichnet, daß es 5 bis 30 Gew.% eines Überzugsmittels nach Anspruch 15 enthält.

**Patentansprüche für folgenden Vertragsstaat: AT**

**1.** Verfahren zur Herstellung von Kondesnsationsprodukten, die im wesentlichen substituierte Propylenharnstoffe der allgemeinen Formeln (I) und/oder (II)

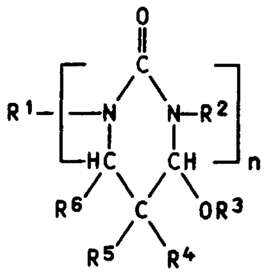

(I)

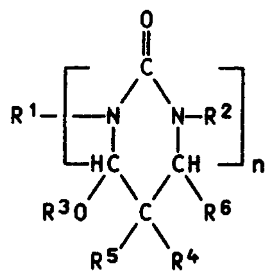

(II)

enthalten, worin $R^1$ einen mindestens zweiwertigen organischen, 4 bis 60 Kohlenstoffatome enthaltenden geradkettigen oder verzweigten Alkylen-, Cycloalkylen-, Oxaalkylen- oder Azaalkylenrest bedeutet, wobei der Rest $R^1$ gegebenenfalls eine oder mehrere Hydroxylgruppen und/oder mit Hydroxyalkyl-, Hydroxycycloalkyl-, Hydroxyoxaalkyl- und/oder Hydroxyazaalkylgruppen substituierte Harnstoff-, Carbamat-, Carbonamid- oder Sulfonamidgruppen enthalten kann,
$R^2$, $R^3$, $R^4$, $R^5$ und $R^6$ untereinander gleich oder verschieden sind und für Wasserstoff, Alkylreste mit 1 bis 18 Kohlenstoffatomen, Cycloalkylreste mit 5 bis 15 Kohlenstoffatomen, Arylreste mit 6 bis 15 Kohlenstoffatomen, Aralkylreste mit 7 bis 16 Kohlenstoffatomen, Oxaalkyl- oder Azaalkylreste mit 2 bis 18 Kohlenstoffatomen, stehen, und $R^2$ zusätzlich für einen Hydroxyalkylrest stehen kann, mit der Maßgabe, daß für den Fall, daß $R^2$ für einen Hydroxyalkylrest steht, dieser 4 bis 18 Kohlenstoffatome enthält, und $R^2$ nicht für einen Aryl- oder Aralkylrest steht,
und n = 2 bis 20 ist, wobei die Wertigkeit von $R^1$ n entspricht und die einzelnen Reste $R^2$ bis $R^6$ der n Propylenharnstoffeinheiten der Formeln (I) und/oder (II) innerhalb der oben angegeben Definitionen untereinander jeweils gleich oder verschieden sein können,
dadurch gekennzeichnet, daß man zunächst

a) primäre Di- und/oder Polyamine der allgemeinen Formel (III)

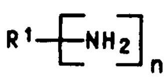

mit

b) Harnstoff, monosubstituierten oder symmetrisch disubstituierten Harnstoffen der allgemeinen Formel (IV) $R^2NH\text{-}CO\text{-}NHR^2$

bei Temperaturen von 100 bis 200 °C, gegebenenfalls in Anwesenheit von sauren oder basischen Katalysatoren sowie gegebenenfalls in Anwesenheit von

c) Dihydroxiverbindungen, Polyhydroxiverbindungen, Alkanolaminen, Hydroxyalkylcarbonsäuren und/oder Hydroxyalkylsulfonsäuren oder Hydroxyalkylsulfonsäureestern

umsetzt und die so erhaltenen Produkte mit

d) 1,4 bis 2,5 Mol eines oder mehrerer Aldehyde der allgemeinen Formel (V)

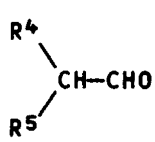 und/oder (VI) $R^6\text{-}CHO$

pro Mol Harnstoff (b) in Gegenwart saurer Katalysatoren bei Temperaturen von 50 bis 150 °C, gegebenenfalls in Anwesenheit eines Lösungsmittels umsetzt, mit der Maßgabe, daß mindestens 0,7 Mol pro Mol eingesetztem Harnstoff (b) eines Aldehyds (d) verwendet werden, der mindestens ein in $\alpha$-Stellung zur Aldehydgruppe befindliches Wasserstoffatom enthält,

oder daß man

a) primäre Monoamine der allgemeinen Formel (VII) $R^2\text{-}NH_2$ oder primäre Di- und/oder Polyamine der allgemeinen Formel (III)

$$R^1\!\left[NH_2\right]_n$$

zunächst mit
e) Monoisocyanaten der allgemeinen Formel (VIII) $R^2$-NCO oder Di- und/oder Polyisocyanaten der allgemeinen Formel (IX)

$$R^1\!\left[NCO\right]_n$$

umsetzt mit der Maßgabe, daß für den Fall, daß als Komponente (a) primäre Monoamine $R^2$-$NH_2$ eingesetzt werden, diese mit Di- und/oder Polyisocyanaten

$$R^1\!\left[NCO\right]_n$$

umsetzt werden, und für den Fall, daß als Komponente (e) Monoisocyanate $R^2$-NCO eingesetzt werden, diese mit Di- und/oder Polyaminen

$$R^1\!\left[NH_2\right]_n$$

umgesetzt werden und die so erhaltenen Di- oder Polyharnstoffe mit
d) 1,4 bis 2,5 Mol eines oder mehrerer Aldehyde

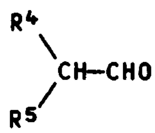 und/oder $R^6$—CHO

pro Mol der bei der Umsetzung von (a) mit (e) gebildeten Harnstoffeinheiten in Gegenwart saurer Katalysatoren bei Temperaturen von 50 bis 150 ° C, gegebenenfalls in Anwesenheit eines Lösungsmittels, umsetzt, mit der Maßgabe, daß mindestens 0,7 Mol pro Mol der durch Umsetezung von a) mit e) gebildeten Harnstoffeinheiten mindestens eines Aldehyds d) verwendet werden, der mindestens ein in α-Stellung zur Aldehydegruppe befindliches Wasserstoffatom enthält.

2. Verfahren nach Anspruch 1, dadurch gekennzeichnet, daß als Aldehyd d) Isobutyraldehyd oder ein Gemisch von Isobutyraldehyd mit Formaldehyd verwendet wird.

3. Verfahren zur Herstellung von Umsetzungsprodukten von Kondensationsprodukten, die im wesentlichen substituierte Propylenharnstoffe der allgemeinen Formeln (I) und/oder (II)

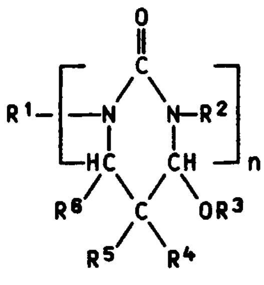

(I)

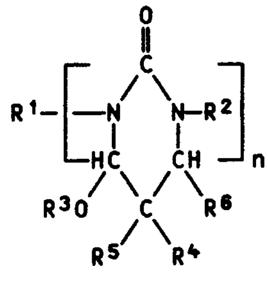

(II)

enthalten, worin $R^1$ einen mindestens zweiwertigen organischen 4 bis 60 Kohlenstoffatome enthaltenden geradkettigen oder verzweigten Alkylen-, Cycloalkylen-, Oxaalkylen- oder Azaalkylenrest bedeutet, wobei der Rest $R^1$ gegebenenfalls eine oder mehrere Hydroxylgruppen und/oder mit Hydroxyalkyl-, Hydroxycycloalkyl-, Hydroxyoxaalkyl- und/oder Hydroxyazaalkylgruppen substituierte Harnstoff-, Carbamat-, Carbonamid- oder Sulfonamidgruppen enthalten kann,
$R^2$, $R^3$, $R^4$, $R^5$ und $R^6$ untereinander gleich oder verschieden sind und für Wasserstoff, Alkylreste mit 1 bis 18 Kohlenstoffatomen, Cycloalkylreste mit 5 bis 15 Kohlenstoffatomen, Arylreste mit 6 bis 15 Kohlenstoffatomen, Aralkylreste mit 7 bis Kohlenstoffatomen, Oxaalkyl- oder Azaalkylreste mit 2 bis 18 Kohlenstoffatomen, stehen und $R^2$ susätzlich für eien Hydroxyalkylrest stehen kann, mit der Maßgabe, daß für den Fall, daß $R_2$ für einen Hydroxyalkylrest steht, dieser 4 bis 18 Kohlenstoffatome enthält, und $R_2$ nicht für einen Aryl- oder Aralkylrest steht,
und n = 2 bis 20 ist, wobei die Wertigkeit von $R^1$ n entspricht und die einzelnen Reste $R^2$ bis $R^6$ der n Propylenharnstoffeinheiten der Formeln (I) und/oder (II) innerhalb der oben angegebenen Definitionen untereinander jeweils gleich oder verschieden sein können, mit
Di- und/oder Polyisocyanaten,
Derivaten von Di- und/oder Polycarbonsäuren oder
p-substituierten Phenolen und/oder deren o-Methylolderivaten, mit der Maßgabe, daß bei Verwendung von o-Methylolphenolen mindestens einer der Reste $R^1$ oder $R^2$ der Formeln (I) und/oder (II) eine Hydroxylgruppe enthält oder der Rest $R^2$ für Wasserstoff steht,
dadurch gekennzeichnet, daß man zunächst

a) primäre Di- und/oder Polyamine der allgemeinen Formel (III)

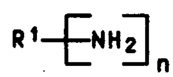

mit

b) Harnstoff, monosubstituierten oder symmetrisch disubstituierten Harnstoffen der allgemeinen Formel (IV) $R^2NH\text{-}CO\text{-}NHR^2$

bei Temperaturen von 100 bis 200 °C, gegebenenfalls in Anwesenheit von sauren oder basischen Katalysatoren sowie gegebenenfalls in Anwesenheit von

c) Dihydroxiverbindungen, Polyhydroxiverbindungen, Alkanolaminen, Hydroxyalkylcarbonsäuren und/oder Hydroxyalkylsulfonsäuren oder Hydroxyalkylsulfonsäureestern

umsetzt und die so erhaltenen Di- und/oder Polyharnstoffe mit

d) 1,4 bis 2,5 Mol eines oder mehrerer Aldehyde der allgemeinen Formel (V)

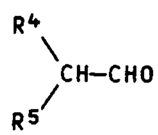

und/oder (VI) $R^6$—CHO

pro Mol Harnstoff (b) in Gegenwart saurer Katalysatoren bei Temperaturen von 50 bis 150 °C, gegebenenfalls in Anwesenheit eines Lösungsmittels umsetzt, mit der Maßgabe, daß mindestens 0,7 Mol pro Mol eingesetztem Harnstoff (b) eines Aldehyds (d) verwent werden, der mindestens ein in $\alpha$-Stellung zur Aldehydgruppe befindliches Wasserstoffatom enthält,

wobei $R^1$ bis $R^6$ und n die in Anspruch 1 angegebene Bedeutung haben, und die so erhaltenen Produkte mit
Di- und/oder Polyisocyanaten,
Derivaten von Di- und/oder Polycarbonsäuren oder
p-substituierten Phenolen und/oder deren o-Methylolderivaten umsetzt, mit der Maßgabe, daß pro Mol eingesetzten Harnstoffs b) 0,25 bis 1,0 Mol Isocyanatgruppen des Di- und/oder Polyisocyanats, Säurehalogenid- und/oder -anhydridgruppen oder p-substituiertes Phenol und/oder dessen o-Methylolderivat verwendet werden,
oder daß man

a) primäre Monoamine der allgemeinen Formel (VII) $R^2$-$NH_2$ oder primäre Di- und/oder Polyamine der allgemeinen Formel (III)

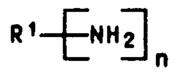

zunächst mit

e) Monoisocyanaten der allgemeinen Formel (VIII) $R^2$-NCO oder Di- und/oder Polyisocyanaten der allgemeinen Formel (IX)

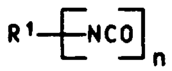

umsetzt mit der Maßgabe, daß für den Fall, daß als Komponente (a) primäre Monoamine $R^2$-$NH_2$ eingesetzt werden, diese mit Di- und/oder Polyisocyanaten

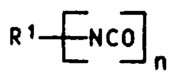

umgesetzt werden, und für den Fall, daß als Komponente (e) Monoisocyanate $R^2$-NCO eingesetzt werden, diese mit Di- und/oder Polyaminen

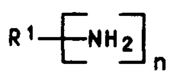

umgesetzt werden und die so erhaltenen Di- oder Polyharnstoffe mit

d) 1,4 bis 2,5 Mol eines oder mehrerer Aldehyde

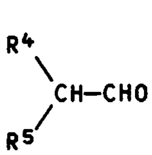 und/oder $R^6$-CHO

pro Mol der bei der Umsetzung von (a) mit (e) gebildeten Harnstoffeinheiten in Gegenwart saurer Katalysatoren bei Temperaturen von 50 bis 150 °C, gegebenenfalls in Anwesenheit eines Lösungsmittels, umsetzt, mit der Maßgabe daß mindestens 0,7 Mol pro Mol der durch Umsetzung von a) mit e) gebildeten Harnstoffeinheiten mindestens eines Aldehyds d) verwendet werden, der mindestens ein in α-Stellung zur Aldehydgruppe befindliches Wasserstoffatom enthält, wobei $R^1$ bis $R^6$ und n die in Anspruch 1 angegebene Bedeutung haben, und die so erhaltenen Produkte mit
Di- und/oder Polyisocyanaten,
Derivaten von Di- und/oder Polycarbonsäuren oder
p-substituierten Phenolen und/oder deren o-Methylolderivaten umsetzt, mit der Maßgabe, daß pro Mol eingesetzten Harnstoffs b) 0,25 bis 1,0 Mol Isocyanatgruppen des Di-und/oder Polyisocyanats, Säurehalogenid und/oder -anhydridgruppen oder p-substituiertes Phenol und/oder dessen o-Methylolderivat verwendet werden.

**4.** Verfahren nach Anspruch 3, dadurch gekennzeichnet, daß als Aldehyd d) Isobutyraldehyd oder ein Gemisch von Isobutyraldehyd mit Formaldehyd verwendet wird.

**5.** Verfahren zur Herstellung eines hitzehärtbaren Überzugsmittels, dadurch gekennzeichnet, daß man als Bindemittel ein Gemisch aus
(A) mindestens einem Polyadditions-, Polykondensations- oder Polymerisationsprodukt mit einem mittleren Molekulargewicht $\overline{M}_n$ von 500 bis 10 000, welches im Mittel pro Molekül mindestens zwei Hydroxylgruppen sowie zusätzlich gegebenenfalls primäre und/oder sekundäre und/oder tertiäre Aminogruppen besitzt und
(B) einem nach einem Verfahren gemäß Anspruch 1 hergestellten Kondensationsprodukt
einsetzt.

**6.** Verfahren zur Herstellung eines hitzehärtbaren Überzugsmittels, dadurch gekennzeichnet, daß man als Bindemittel ein Gemisch aus
(A) mindestens einem Polyadditions-, Polykondensations- oder Polymerisationsprodukt mit einem mittleren Molekulargewicht $\overline{M}_n$ von 500 bis 10 000, welches im Mittel pro Molekül mindestens zwei Hydroxylgruppen sowie zusätzlich gegebenenfalls primäre und/oder sekundäre und/oder tertiäre Aminogruppen besitzt und
(B) einem nach einem Verfahren gemäß Anspruch 3 hergestellten Umsetzungsprodukt
einsetzt.

**7.** Verfahren nach Anspruch 5 oder 6, dadurch gekennzeichnet, daß man als Komponente (A) ein Polyesterharz einsetzt.

**8.** Verfahren nach Anspruch 5 oder 6, dadurch gekennzeichnet, daß man als Komponente (A) ein Umsetzungsprodukt eines Epoxidharzes mit mindestens einem Amin, Alkohol oder Mercaptan einsetzt.

**9.** Verfahren nach Anspruch 5 oder 6, dadurch gekennzeichnet, daß man als Komponente (A) ein hydroxylgruppenhaltiges Polyacrylatharz einsetzt.

**10.** Verfahren zur Herstellung wäßriger Einbrennlacke und kathodisch abscheidbarer Elektrotauchlacke, dadurch gekennzeichnet, daß die nach einem Verfahren gemäß einem der Ansprüche 5 bis 9 hergestellten hitzehärtbaren Überzugsmittel durch zumindest teilweise Neutralisation mit einer Säure wasserverdünnbar gemacht werden.

**Claims**
**Claims for the following Contracting States : DE, FR, GB, IT, NL, SE**

**1.** A reaction product of condensates which essentially contain substituted propyleneureas of the formulae (I) and/or (II)

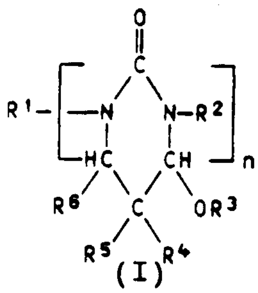
(I)

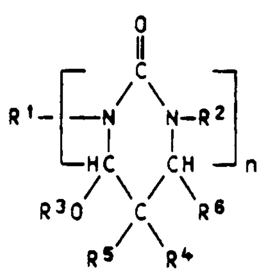
(II)

where $R^1$ is a divalent or polyvalent, straight-chain or branched alkylene, cycloalkylene, oxaalkylene or azaalkylene radical of 4 to 60 carbon atoms which may contain one or more hydroxyl groups and/or urea, carbamate, carboxamide or sulfonamide groups substituted by hydroxyalkyl, hydroxycycloalkyl, hydroxyoxaalkyl and/or hydroxyazaalkyl, $R^2$, $R^3$, $R^4$, $R^5$ and $R^6$ are identical or different and are each

hydrogen, alkyl of 1 to 18 carbon atoms, cycloalkyl of 5 to 15 carbon atoms, aryl of 6 to 15 carbon atoms, aralkyl of 7 to 16 carbon atoms or oxaalkyl or azaalkyl, each of 2 to 18 carbon atoms, and $R^2$ may furthermore be hydroxyalkyl, with the proviso that where $R^2$ is hydroxyalkyl the latter is of 4 to 18 carbon atoms and the other radical or radicals $R^2$ are not aryl or aralkyl, and n is from 2 to 20, and the valency of $R^1$ corresponds to n and the individual radicals $R^2$, $R^3$, $R^4$, $R^5$ and $R^6$ of the n propyleneurea units of the formulae (I) and/or (II) may be identical or different while conforming to the stated definitions, with

di- and/or polyisocyanates,

derivatives of di- and/or polycarboxylic acids or p-substituted phenols and/or their o-methylol derivatives, with the proviso that, where o-methylolphenols are used, one or more of the radicals $R^1$ and $R^2$ of the formulae (I) and/or (II) contain a hydroxyl group or $R^2$ is hydrogen.

**2.** A condensate which essentially contains substituted propyleneureas of the formulae (I) and/or (II)

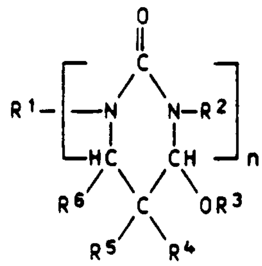

(I)

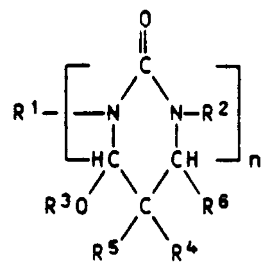

(II)

where $R^1$ is a divalent or polyvalent, straight-chain or branched alkylene, cycloalkylene, oxaalkylene or azaalkylene radical of 4 to 60 carbon atoms which may contain one or more hydroxyl groups and/or urea, carbamate, carboxamide or sulfonamide groups substituted by hydroxyalkyl, hydroxycycloalkyl, hydroxyoxaalkyl and/or hydroxyazaalkyl, $R^2$, $R^3$, $R^4$, $R^5$ and $R^6$ are identical or different and are each hydrogen, alkyl or 1 to 18 carbon atoms, cycloalkyl of 5 to 15 carbon atoms, aryl of 6 to 15 carbon atoms, aralkyl of 7 to 16 carbon atoms or oxaalkyl or azaalkyl, each of 2 to 18 carbon atoms, and $R^2$ may furthermore be hydroxyalkyl, with the proviso that where $R^2$ is hydroxyalkyl the latter is of 4 to 18 carbon atoms and the other radical or radicals $R^2$ are not aryl or aralkyl, and n is from 2 to 20, and the valency of $R^1$ corresponds to n and the individual radicals $R^2$, $R^3$, $R^4$, $R^5$ and $R^6$ of the n propyleneurea units of the formulae (I) and/or (II) may be identical or different while conforming to the stated definitions.

**3.** A process for the preparation of a condensate as claimed in claim 2, wherein first

a) primary di- and/or polyamines of the formula (III)

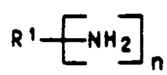

are reacted with

b) urea or monosubstituted or symmetrically disubstituted ureas of the formula (IV) $R^2NH\text{-}CO\text{-}NHR^2$

at from 100 to 200 ° C, in the presence or absence of an acidic or basic catalyst and of

c) dihydroxy compounds, polyhydroxy compounds, alkanolamines, hydroxyalkylcarboxylic acids and/or hydroxyalkylsulfonic acids or hydroxyalkylsulfonates,

and the resulting products are reacted with

d) from 1.4 to 2.5 moles of one or more aldehydes of the formula (V)

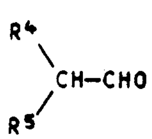

and/or (VI) $R^6\text{-}CHO$

per mole of urea (b) in the presence of an acidic catalyst at from 50 to 150 ° C, in the presence or absence of a solvent, with the proviso that not less than 0.7 mole of an aldehyde (d) which contains one or more hydrogen atoms α to the aldehyde group is used per mole of urea (b) employed,
$R^1$ to $R^6$ and n having the meanings stated in claim 2.

**4.** A process for the preparation of a condensate as claimed in claim 2 wherein
a) primary monoamines of the formula (VII), $R^2$-$NH_2$, or primary di- and/or polyamines of the formula (III)

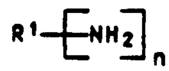

are first reacted with
e) monoisocyanates of the formula (VIII), $R^2$-NCO, or di- and/or polyisocyanates of the formual (IX)

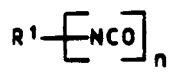

with the proviso that, where a primary monoamine $R^2$-$NH_2$ is used as component (a), this is reacted with di- and/or polyisocyanates

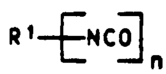

and, where a monoisocyanate $R^2$-NCO is used as component (e), this is reacted with di- and/or polyamines

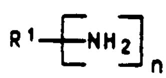

and the resulting di- or polyureas are reacted with
d) from 1.4 to 2.5 moles of one or more aldehydes

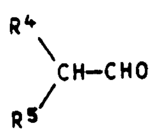 and/or $R^6$-CHO

per mole of the urea units formed in the reaction of (a) with (e), in the presence of an acidic catalyst at from 50 to 150 ° C and in the presence or absence of a solvent, with the proviso that not less than 0.7 mole of one or more aldehydes d) which contain one or more hydrogen atoms α to the aldehyde group is used per mole of the urea units formed by reacting a) with e),
$R^1$ to $R^6$ and n having the meanings stated in claim 2.

**5.** A process as claimed in claim 4, wherein isobutyraldehyde or a mixture of isobutyraldehyde with formaldehyde is used as the aldehyde d).

**6.** A process for the preparation of a reaction product as claimed in claim 1, wherein
a) primary di- and/or polyamines of the formula (III)

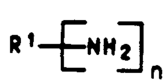

are first reacted with

b) urea or monosubstituted or symmetrically disubstituted ureas of the formula (IV) $R^2NH\text{-}CO\text{-}NHR^2$ at from 100 to 200 °C, in the presence or absence of an acidic or basic catalyst and of

c) dihydroxy compounds, polyhydroxy compounds, alkanolamines, hydroxyalkylcarboxylic acids and/or hydroxyalkylsulfonic acids or hydroxyalkylsulfonates,

and the resulting di- and/or polyureas are reacted with

d) from 1.4 to 2.5 moles of one or more aldehydes of the formula (V)

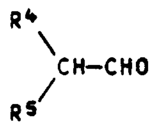

and/or $R^6CHO$

per mole of urea (b) in the presence of an acidic catalyst at from 50 to 150 °C, in the presence or absence of a solvent, with the proviso that not less than 0.7 mole of an aldehyde (d) which contains one or more hydrogen atoms $\alpha$ to the aldehyde group is used per mole of urea (b) employed,

$R^1$ to $R^6$ and n having the meanings stated in claim 1, and the products thus obtained are reacted with di- and/or polyisocyanates,

derivatives of di- and/or polycarboxylic acids or

p-substituted phenols and/or their o-methylol derivatives, with the proviso that from 0.25 to 1.0 mole of isocyanate groups of the di- and/or polyisocyanate, acyl halide and/or anhydride groups or p-substituted phenol and/or its o-methylol derivative is used per mole of urea b) employed.

**7.** A process for the preparation of reaction product as claimed in claim 1, wherein

a) primary monoamines of the formula (VII), $R^2\text{-}NH_2$, or primary di- and/or polyamines of the formula (III)

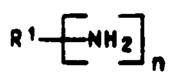

are first reacted with

e) monoisocyanates of the formula (VIII), $R^2\text{-}NCO$, or di- and/or polyisocyanates of the formula (IX)

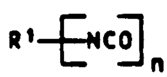

with the proviso that, where a primary monoamine $R^2\text{-}NH_2$ is used as component (a), this is reacted with di- and/or polyisocyanates

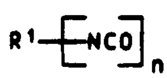

and, where a monoisocyanate $R^2\text{-}NCO$ is used as component (e), this is reacted with di- and/or polyamines

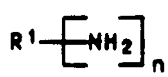

and the resulting di- or polyureas are reacted with

d) from 1.4 to 2.5 moles of one or more aldehydes

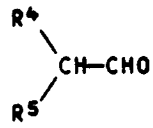 and/or $R^6$-CHO

per mole of the urea units formed in the reaction of (a) with (e), in the presence of an acidic catalyst at from 50 to 150 ° C and in the presence or absence of a solvent, with the proviso that not less than 0.7 mole of one or more aldehydes d) which contains one or more hydrogen atoms $\alpha$ to the aldehyde group is used per mole of the urea units formed by reacting a) with e),

$R^1$ to $R^6$ and n having the meanings stated in claim 1, and the products thus obtained are reacted with di- and/or polyisocyanates,
derivatives of di- and/or polycarboxylic acids or
p-substituted phenols and/or their o-methylol derivatives, with the proviso that from 0.25 to 1.0 mole of isocyanate groups of the di- and/or polyisocyanate, acyl halide and/or anhydride groups or p-substituted phenol and/or its o-methylol derivative is used per mole of urea b) employed.

**8.** A process as claimed in claim 7, wherein isobutyraldehyde or a mixture of isobutyraldehyde with formaldehyde is used as the aldehyde d).

**9.** A heat-curable coating material, which contains, as a binder, a mixture of
(A) one or more polyadducts, polycondensates or polymers which have a mean molecular weight $\overline{M}_n$ of from 500 to 10,000 and possess on average two or more hydroxyl groups per molecule and may additionally possess primary and/or secondary and/or tertiary amino groups and
(B) a reaction product as claimed in claim 1.

**10.** A heat-curable coating material, which contains, as the binder, a mixture of
(A) one or more polyadducts, polycondensates or polymers which have a mean molecular weight $\overline{M}_n$ of from 500 to 10,000 and possess on average two or more hydroxyl groups per molecule and may additionally possess primary and/or secondary and/or tertiary amino groups and
(B) a condensate as claimed in claim 2.

**11.** A coating material as claimed in claim 9 or 10, wherein component (A) is a polyester resin.

**12.** A coating material as claimed in claim 9 or 10, wherein component (A) is a reaction product of an epoxy resin with one or more amines, alcohols or mercaptans.

**13.** A coating material as claimed in claim 9 or 10, wherein component (A) is a hydroxyl-containing polyacrylate resin.

**14.** Use of a coating material as claimed in any of claims 9 to 13 for solvent-containing baking finishes.

**15.** A coating material as claimed in any of claims 9 to 13, which is rendered water-dilutable by partial or complete neutralization with an acid.

**16.** Use of a coating material as claimed in claim 15 for the preparation of cathodic electrocoating finishes.

**17.** Use of a coating material as claimed in claim 15 for aqueous baking finishes.

**18.** An aqueous cathodic electrocoating bath, which contains from 5 to 30% by weight of a coating material as claimed in claim 15.

**Claims for the following Contracting State : AT**

**1.** A process for the preparation of a condensate which essentially contains substituted propyleneureas of the formulae (I) and/or (II)

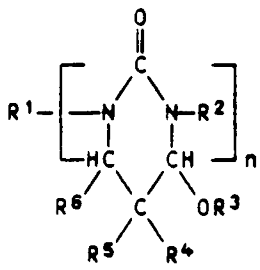

(I)

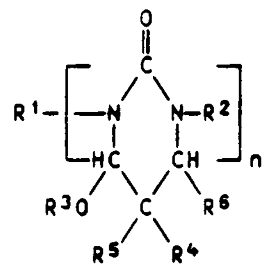

(II)

where $R^1$ is a divalent or polyvalent, straight-chain or branched alkylene, cycloalkylene, oxoaalkylene or azaalkylene radical of 4 to 60 carbon atoms which may contain one or more hydroxyl groups and/or urea, carbamate, carboxamide or sulfonamide groups substituted by hydroxyalkyl, hydroxycycloalkyl, hydroxyoxaalkyl and/or hydroxyazaalkyl, $R^2$, $R^3$, $R^4$, $R^5$, and $R^6$ are identical or different and are each hydrogen, alkyl of 1 to 18 carbon atoms, cycloalkyl of 5 to 15 carbon atoms, aryl of 6 to 15 carbon atoms, aralkyl of 7 to 16 carbon atoms or oxaalkyl or azaalkyl, each of 2 to 18 carbon atoms, and $R^2$ may furthermore be hydroxyalkyl, with the proviso that where $R^2$ is hydroxyalkyl the latter is of 4 to 18 carbon atoms and the other radical or radicals $R^2$ are not aryl or aralkyl, and n is from 2 to 20, and the valency of $R^1$ corresponds to n and the individual radicals $R^2$, $R^3$, $R^4$, $R^5$ and $R^6$ of the n propyleneurea units of the formulae (I) and/or (II) may be identical or different while conforming to the stated definitions, wherein first

a) primary di- and/or polyamines of the formula (III)

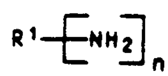

are reacted with

b) urea or monosubstituted or symmetrically disubstituted ureas of the formula (IV) $R^2NH\text{-}CO\text{-}NHR^2$

at from 100 to 200 °C, in the presence or absence of an acidic or basic catalyst and of

c) dihydroxy compounds, polyhydroxy compounds, alkanolamines, hydroxyalkylcarboxylic acids and/or hydroxyalkylsulfonic acids or hydroxyalkylsulfonates,

and the resulting products are reacted with

d) from 1.4 to 2.5 moles of one or more aldehydes of the formula (V)

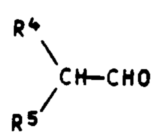

and/or (VI) $R^6\text{—}CHO$

per mole of urea (b) in the presence of an acidic catalyst at from 50 to 150 °C, in the presence or absence of a solvent, with the proviso that not less than 0.7 mole of an aldehyde (d) which contains one or more hydrogen atoms $\alpha$ to the aldehyde group is used per mole of urea (b) employed,

or wherein

a) primary monoamines of the formula (VII), $R^2\text{-}NH_2$, or primary di- and/or polyamines of the formula (III)

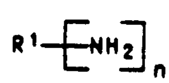

are first reacted with

e) monoisocyanates of the formula (VIII), $R^2\text{-}NCO$, or di- and/or polyisocyanates of the formula (IX)

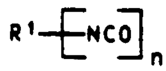

with the proviso that, where a primary monoamine $R^2$-$NH_2$ is used as component (a), this is reacted with di- and/or polyisocyanates

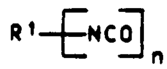

and, where a monoisocyanate $R^2$-NCO is used as component (e), this is reacted with di- and/or polyamines

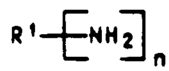

and the resulting di- or polyureas are reacted with
d) from 1.4 to 2.5 moles of one or more aldehydes

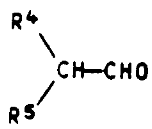 and/or $R^6$–CHO

per mole of the urea units formed in the reaction of (a) with (e), in the presence of an acidic catalyst at from 50 to 150 ° C and in the presence or absence of a solvent, with the proviso that not less than 0.7 mole of one or more aldehydes d) which contain one or more hydrogen atoms $\alpha$ to the aldehyde group is used per mole of the urea units formed by reacting a) with e).

2. A process as claimed in claim 1, wherein isobutyraldehyde or a mixture of isobutyraldehyde with formaldehyde is used as the aldehyde d).

3. A process for the preparation of a reaction product of condensates which essentially contain substituted propyleneureas of the formulae (I) and/or (II)

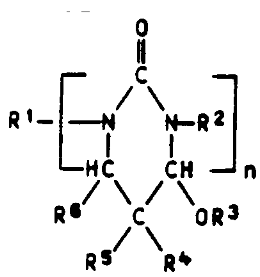
(I)

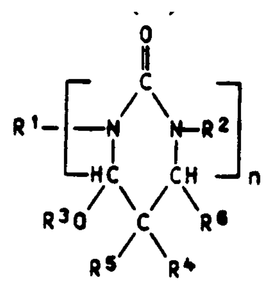
(II)

where $R^1$ is a divalent or polyvalent, straight-chain or branched alkylene, cycloalkylene, oxaalkylene or azaalkylene radical of 4 to 60 carbon atoms which may contain one or more hydroxyl groups and/or urea, carbamate, carboxamide or sulfonamide groups substituted by hydroxyalkyl, hydroxycycloalkyl, hydroxyoxaalky and/or hydroxyazaalkyl, $R^2$, $R^3$, $R^4$, $R^5$, and $R^6$ are identical or different and are each hydrogen, alkyl of 1 to 18 carbon atoms, cycloalkyl of 5 to 15 carbon atoms, aryl of 6 to 15 carbon atoms, aralkyl of 7 to 16 carbon atoms or oxaalkyl or azaalkyl, each of 2 to 18 carbon atoms, and $R^2$ may furthermore by hydroxyalkyl, with the proviso that where $R^2$ is hydroxyalkyl the latter is of 4 to 18 carbon atoms and the other radical or radicals $R^2$ are not aryl or aralkyl, and n is from 2 to 20, and the valency of $R^1$ corresponds to n and the individual radicals $R^2$, $R^3$, $R^4$, $R^5$ and $R^6$ of the n propyleneurea

units of the formulae (I) and/or (II) may be identical or different while conforming to the stated definitions, with
di- and/or polyisocyanates,
derivatives of di- and/or polycarboxylic acids or p-substituted phenols and/or their o-methylol derivatives, with the proviso that, where o-methylolphenols are used, one or more of the radicals $R^1$ and $R^2$ of the formulae (I) and/or (II) contain a hydroxyl group or $R^2$ is hydrogen,
wherein,

a) primary di- and/or polyamines of the formula (III)

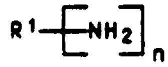

are first reacted with

b) urea or monosubstituted or symmetrically disubstituted ureas of the formula (IV) $R^2NH\text{-}CO\text{-}NHR^2$
at from 100 to 200 ° C, in the presence or absence of an acidic or basic catalyst and of

C) dihydroxy compounds, polyhydroxy compounds, alkanolamines, hydroxyalkylcarboxylic acids and/or hydroxyalkylsulfonic acids or hydroxyalkylsulfonates,

and the resulting di- and/or polyureas are reacted with

d) from 1.4 to 2.5 moles of one or more aldehydes of the formula (v)

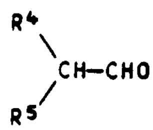 and/or (VI) 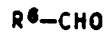

per mole of urea (b) in the presence of an acidic catalyst at from 50 to 150 ° C, in the presence or absence of a solvent, with the proviso that not less than 0.7 mole of an aldehyde (d) which contains one or more hydrogen atoms $\alpha$ to the aldehyde group is used per mole of urea (b) employed,

$R^1$ to $R^6$ and n having the meanings stated in claim 1, and the products thus obtained are reacted with di- and/or polyisocyanates, derivatives of di- and/or polycarboxylic acids or p-substituted phenols and/or their o-methylol derivatives, with the proviso that from 0.25 to 1.0 mole of isocyanate groups of the di- and/or polyisocyanate, acyl halide and/or anhydride groups or p-substituted phenol and/or its o-methylol derivative is used per mole of urea b) employed,
or wherein

a) primary monoamines of the formula (VII), $R^2\text{-}NH_2$, or primary di- and/or polyamines of the formula (III)

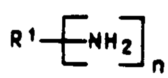

are first reacted with

e) monoisocyanates of the formula (VIII), $R^2\text{-}NCO$, or di- and/or polyisocyanates of the formula (IX)

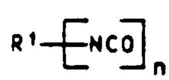

with the proviso that, where a primary monoamine $R^2\text{-}NH_2$ is used as component (a), this is reacted with di- and/or polyisocyanates

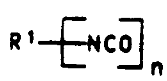

and, where a nonoisocyanate $R^2\text{-}NCO$ is used as component (e), this is reacted with di- and/or

polyamines

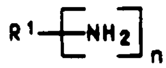

and the resulting di- or polyureas are reacted with
d) from 1.4 to 2.5 moles of one or more aldehydes

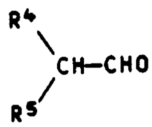 and/or 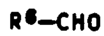

per mole of the urea units formed in the reaction of (a) with (e), in the presence of an acidic catalyst at from 50 to 150 ° C and in the presence or absence of a solvent, with the proviso that not less than 0.7 mole of one or more aldehydes d) which contains one or more hydrogen atoms $\alpha$ to the aldehyde group is used per mole of the urea units formed by reacting a) with e),

$R^1$ to $R^5$ and n having the meanings stated in claim 1, and the products thus obtained are reacted with
di- and/or polyisocyanates,
derivatives of di- and/or polycarboxylic acids or
p-substituted phenols and/or their o-methylol derivatives, with the proviso that from 0.25 to 1.0 mole of isocyanate groups of the di- and/or polyisocyanate, acyl halide and/or anhydride groups or p-substituted phenol and/or its o-methylol derivative is used per mole of urea b) employed.

**4.** A process as claimed in claim 3, wherein isobutyraldehyde or a mixture of isobutyraldehyde with formaldehyde is used as the aldehyde d).

**5.** A process for the preparation of a heat-curable coating material, in which a mixture of
(A) one or more polyadducts, polycondensates or polymers which have a mean molecular weight $\overline{M}_n$ of from 500 to 10,000 and possess on average two or more hydroxyl groups per molecule and may additionally possess primary and/or secondary and/or tertiary amino groups and
(B) a condensate prepared by a method as claimed in claim 1, is used as the binder.

**6.** A process for the preparation of a heat-curable coating material, in which a mixture of
(A) one or more polyadducts, polycondensates or polymers which have a mean molecular weight $\overline{M}_n$ of from 500 to 10,000 and possess on average two or more hydroxyl groups per molecule and may additionally possess primary and/or secondary and/or tertiary amino groups and
(B) a reaction product prepared by a process as claimed in claim 3, is used as the binder.

**7.** A process as claimed in claim 5 or 6, wherein a polyester resin is used as component (A).

**8.** A process as claimed in claim 5 or 6, wherein a reaction product of an epoxy resin with one or more amines, alcohols or mercaptans is used as component (A).

**9.** A process as claimed in claim 5 or 6, wherein a hydroxyl-containing polyacrylate resin is used as component (A).

**10.** A process for the preparation of an aqueous baking finish or a cathodic electrocoating finish, wherein a heat-curable coating material prepared by a process as claimed in any of claims 5 to 9 is rendered water-dilutable by partial or complete neutralization with an acid.

**Revendications**

**Revendications pour les Etats contractants suivants : DE, FR, GB, IT, NL, SE**

**1.** Produits de réaction de produits de condensation qui contiennent essentiellement des propylène-urées substituées de formules générales (I) et/ou (II)

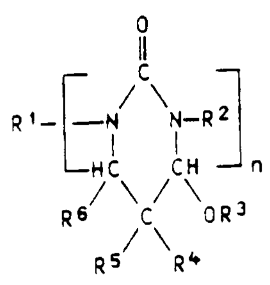

(I)

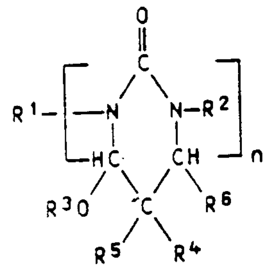

(II)

dans lesquelles $R^1$ représente un reste organique au moins bivalent alkylène, cycloalkylène, oxaalkylène ou azaalkylène, en chaîne droite ou ramifiée, contenant 4 à 60 atomes de carbone, le reste $R^1$ pouvant contenir éventuellement un ou plusieurs groupements hydroxyle et/ou groupements urée, carbamate, carbonamide ou sulfonamide substitués par des groupements hydroxyalkyle, hydroxycycloalkyle, hydroxyoxaalkyle et/ou hydroxyazaalkyle,
$R^2$, $R^3$, $R^4$, $R^5$ et $R^6$ sont identiques ou différents les uns des autres et sont mis pour des atomes d'hydrogène, des restes alkyle à 1-18 atomes de carbone, des restes cycloalkyle à 5-15 atomes de carbone, des restes aryle à 6-15 atomes de carbone, des restes aralkyle à 7-16 atomes de carbone ou des restes oxaalkyle ou azaalkyle à 2-13 atomes de carbone, $R^2$ peut en plus être mis pour un reste hydroxyalkyle, étant spécifié que dans le cas où $R^2$ est mis pour un reste hydroxyalkyle, celui-ci contient de 4 à 18 atomes de carbone, et $R^2$ n'est pas mis pour un reste aryle ou aralkyle,
et n = 2 à 20, la valence de $R^1$ correspondant à n et les restes $R^2$ à $R^6$ individuels des n motifs propylène-urée des formules (I) et/ou (II) pouvant être chaque fois identiques ou différents les uns des autres dans le cadre des définitions données ci-dessus,
avec
des di- et/ou polyisocyanates,
des dérivés d'acides di- et/ou polycarboxyliques ou des phénols p-substitués et/ou leurs dérivés o-méthylolés, étant spécifié qu'en cas d'utilisation d'o-méthylolphénols, l'un au moins des restes $R^1$ ou $R^2$ des formules (I) et/ou (II) contient un groupement hydroxyle ou le reste $R^2$ est mis pour un atome d'hydrogène.

**2.** Produits de condensation qui contiennent essentiellement des propylène-urées substituées de formules générales (I) et/ou (II)

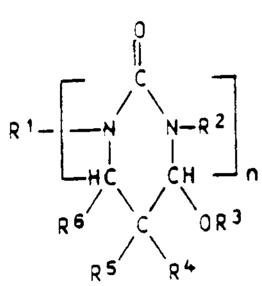

(I)

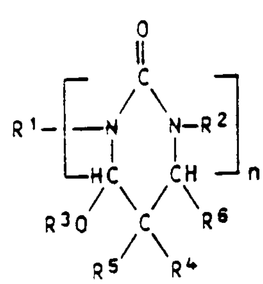

(II)

dans lesquelles $R^1$ represente un reste organique au moins bivalent alkylène, cycloalkylène, oxaalkylène ou azaalkylène, en chaîne droite ou ramifiée, contenant 4 à 60 atomes de carbone, le reste $R^1$ pouvant contenir éventuellement un ou plusieurs groupements hydroxyle et/ou groupements urée, carbamate, carbonamide ou sulfonamide substitués par des groupements hydroxyalkyle, hydroxycycloalkyle, hydroxyoxaalkyle et/ou hydroxyazaalkyle,
$R^2$, $R^3$, $R^4$, $R^5$ et $R^6$ sont identiques ou différents les uns des autres et sont mis pour des atomes d'hydrogène, des restes alkyle à 1-13 atomes de carbone, des restes cycloalkyle à 5-15 atomes de carbone, des restes aryle à 6-15 atomes de carbone, des restes aralkyle à 7-16 atomes de carbone ou des restes oxaalkyle ou azaalkyle à 2-18 atomes de carbone, $R^2$ peut en plus être mis pour un reste

hydroxyalkyle, étant spécifié que dans le cas où $R^2$ est mis pour un reste hydroxyalkyle, celui-ci contient de 4 à 18 atomes de carbone, et $R^2$ n'est pas mis pour un reste aryle ou aralkyle,
et n = 2 à 20, la valence de $R^1$ correspondant à n et les restes $R^2$ à $R^6$ individuels des n motifs propylène-urée des formules (I) et/ou (II) pouvant être chaque fois identiques ou différents les uns des autres dans le cadre des définitions données ci-dessus.

**3.** Procédé de préparation des produits de condensation selon la revendication 2, caractérisé en ce qu'on fait d'abord réagir
a) des di- et/ou polyamines primaires de formule générale (III)

$R^1 [NH_2]_n$

avec
b) de l'urée, des urées monosubstituées ou disubstituées symétriques de formule générale (IV) $R^2NH\text{-}CO\text{-}NHR^2$,
à des températures de 100 à 200 °C, éventuellement en présence de catalyseurs acides ou basiques et éventuellement en présence
c) de composés dihydroxylés, de composés polyhydroxylés, d'alcanolamines, d'acides hydroxyalkylcarboxyliques et/ou d'acides hydroxyalkylsulfoniques ou d'esters d'acides hydroxyalkylsulfoniques,
et on fait réagir les produits ainsi obtenus avec
d) 1,4 à 2,5 moles d'un ou de plusieurs aldéhydes de formule générale (V)

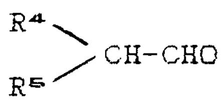 et/ou (VI) $R^6$-CHO

par mode d'urée (b), en présence de catalyseurs acides à des températures de 50 à 150 °C, éventuellement en présence d'un solvant, étant spécifié qu'on utilise, par mole d'urée (b) mise en réaction, au moins 0,7 mole d'un aldéhyde (d) qui contient au moins un atome d'hydrogène se trouvant en position $\alpha$ par rapport au groupement aldéhyde,
$R^1$ a $R^6$ et n ayant les significations indiquées dans la revendication 2.

**4.** Procédé de préparation des produits de condensation selon la revendication 2, caractérisé en ce qu'on fait d'abord réagir
a) des monoamines primaires de formule générale (VII) $R^2\text{-}NH_2$ ou des di- et/ou polyamines primaires de formule générale (III)

$R^1 [NH_2]_n$

avec
e) des monoisocyanates de formule générale (VIII) $R^2$-NCO ou des di- et/ou polyisocyanates de formule générale (IX)

$R^1 [NCO]_n$

étant specifié que dans le cas où des monoamines primaires $R^2\text{-}NH_2$ sont utilisées comme composant (a), elles sont mises en reaction avec des di- et/ou polyisocyanates

$R^1 [NCO]_n$

et que dans le cas où des monoisocyanates $R^2$-NCO sont utilisés comme composant (e), ils sont mis en réaction avec des di- et/ou des polyamines

$R^1 [NH_2]_n$

et on fait réagir les di- ou polyurées ainsi obtenues avec
d) 1,4 à 2,5 moles d'un ou de plusieurs aldéhydes

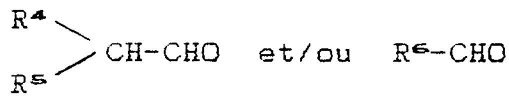

par mole des motifs urée formés par la réaction de (a) avec (e), en présence de catalyseurs acides à des températures de 50 à 150 ° C, éventuellement en présence d'un solvant, étant spécifié qu'on utilise, par mole de motifs urée formés par réaction de (a) avec (e), au moins 0,7 mode d'au moins un aldéhyde (d) qui contient au moins un atome d'hydrogène se trouvant en position $\alpha$ par rapport au groupement aldéhyde,

$R^1$ à $R^6$ et n ayant les significations indiquées dans la revendication 2.

**5.** Procédé selon la revendication 4, caractérisé en ce qu'on utilise, comme aldéhyde (d), de l'isobutyraldéhyde ou un mélange d'isobutyraldéhyde et de formaldéhyde.

**6.** Procédé de préparation des produits de réaction selon la revendication 1, caractérisé en ce qu'on fait d'abord réagir

a) des di- et/ou polyamines primaires de formule générale (III)

$R^1[NH_2]_n$

avec

b) de l'urée, des urées monosubstituées ou disubstituées symétriques de formule générale (IV) $R^2NH\text{-}CO\text{-}NHR^2$,

à des températures de 100 à 200 ° C, éventuellement en présence de catalyseurs acides ou basiques et éventuellement en présence

c) de composés dihydroxylés, de composés polyhydroxylés, d'alcanolamines, d'acides hydroxyalkylcarboxyliques et/ou d'acides hydroxyalkylsulfoniques ou d'esters d'acides hydroxyalkylsulfoniques,

et on fait réagir les di- et/ou polyurées ainsi obtenues avec

d) 1,4 à 2,5 modes d'un ou de plusieurs aldéhydes de formule générale (V)

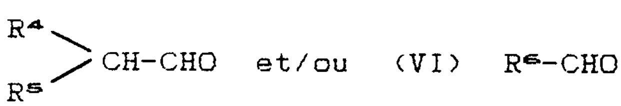

par mole d'urée (b), en présence de catalyseurs acides à des températures de 50 à 150 ° C, éventuellement en présence d'un solvant, étant spécifié qu'on utilise, par mole d'urée (b) mise en réaction, au moins 0,7 mode d'un aldéhyde (d) qui contient au moins un atome d'hydrogène se trouvant en position $\alpha$ par rapport au groupement aldéhyde,

$R^1$ à $R^6$ et n ayant les significations indiquées dans la revendication 1,

et on fait réagir les produits ainsi obtenus avec

des di- et/ou polyisocyanates,

des dérives d'acides di- et/ou polycarboxyliques ou

des phénols p-substitués et/ou leurs dérivés o-méthylolés, étant spécifié qu'on utilise, par mole d'urée (b) mise en réaction, de 0,25 à 1,0 mole de groupements isocyanate du di- et/ou polyisocyanate, de groupements halogénure et/ou anhydride d'acide ou de phénol p-substitué et/ou du dérivé o-méthylolé de celui-ci.

**7.** Procédé de préparation des produits de réaction selon la revendication 1, caractérisé en ce qu'on fait d'abord réagir

a) des monoamines primaires de formule générale (VII) $R^2\text{-}NH_2$ ou des di- et/ou polyamines primaires de formule générale (III)

$R^1[NH_2]_n$

avec

e) des monoisocyanates de formule générale (VIII) $R^2\text{-}NCO$ ou des di- et/ou polyisocyanates de

formule générale (IX)

$R^1[NCO]_n$

étant spécifié que dans le cas où des monoamines primaires $R^2$-$NH_2$ sont utilisées comme composant (a), elles sont mises en réaction avec des di- et/ou polyisocyanates

$R^1[NCO]_n$

et que dans le cas où des monoisocyanates $R^2$-NCO sont utilisés comme composant (e), ils sont mis en réaction avec des di- et/ou des polyamines

$R^1[NH_2]_n$

et on fait réagir les di- ou polyurées ainsi obtenues avec
d) 1,4 à 2,5 moles d'un ou de plusieurs aldéhydes

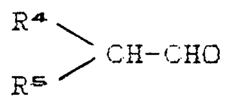 et/ou $R^6$-CHO

par mole des motifs urée formés par la réaction de (a) avec (e), en présence de catalyseurs acides à des températures de 50 à 150 °C, éventuellement en présence d'un solvant, étant spécifié qu'on utilise, par mode de motifs urée formes par reaction de (a) avec (e), au moins 0,7 mole d'au moins un aldéhyde (d) qui contient au moins un atome d'hydrogène se trouvant en position $\alpha$ par rapport au groupement aldéhyde,
$R^1$ à $R^6$ et n ayant les significations indiquées dans la revendication 1,
et on fait réagir les produits ainsi obtenus avec
des di- et/ou polyisocyanates,
des dérivés d'acides di- et/ou polycarboxyliques ou
des phénols p-substitués et/ou leurs dérivés o-méthylolés, étant spécifié qu'on utilise, par mole d'urée (b) mise en réaction, de 0,25 à 1,0 mole de groupements isocyanate du di- et/ou polyisocyanate, de groupements halogénure et/ou anhydride d'acide ou de phénol p-substitué et/ou du dérivé o-méthylolé de celui-ci.

**8.** Procédé selon la revendication 7, caractérisé en ce qu'on utilise, comme aldéhyde (d), de l'isobutyraldéhyde ou un mélange d'isobutyraldéhyde et de formaldéhyde.

**9.** Produits de revêtement thermodurcissables, caractérisés en ce qu'ils contiennent, en tant que liant, un mélange
(A) d'au moins un produit de polyaddition, de polycondensation ou de polymérisation ayant un poids moléculaire moyen $\overline{M}_n$ de 500 à 10 000, qui renferme en moyenne, par molécule, au moins deux groupements hydroxyle et éventuellement et en plus des groupements amino primaires et/ou secondaires et/ou tertiaires, et
(B) d'un produit de réaction selon la revendication 1.

**10.** Produits de revêtement thermodurcissables, caractérisés en ce qu'ils contiennent, en tant que liant, un mélange
(A) d'au moins un produit de polyaddition, de polycondensation ou de polymérisation ayant un poids moléculaire moyen $\overline{M}_n$ de 500 à 10 000, qui renferme en moyenne, par molécule, au moins deux groupements hydroxyle et éventuellement et en plus des groupements amino primaires et/ou secondaires et/ou tertiaires, et
(B) d'un produit de condensation selon la revendication 2.

**11.** Produits de revêtement selon la revendication 9 ou 10, caractérisés en ce que le composent (A) est une résine de polyester.

**12.** Produits de revêtement selon la revendication 9 ou 10, caractérisés en ce que le composant (A) est un produit de réaction d'une résine époxy avec au moins une amine, un alcool ou un mercaptan.

**13.** Produits de revêtement selon la revendication 9 ou 10, caractérisés en ce que le composent (A) est une résine polyacrylique contenant des groupements hydroxyle.

**14.** Utilisation des produits de revêtement selon l'une quelconque des revendications 9 à 13 pour des vernis à cuire contenant un solvant.

**15.** Produits de revêtement selon l'une quelconque des revendications 9 à 13, caractérisés en ce qu'ils sont diluables à l'eau après une neutralisation au moins partielle par un acide.

**16.** Utilisation des produits de revêtement selon la revendication 15 pour la préparation de vernis de trempage électrophorétique à dépôt cathodique.

**17.** Utilisation des produits de revêtement selon la revendication 15 pour des vernis à cuire aqueux.

**18.** Bain de vernis aqueux pour le trempage électrophorétique cathodique, caractérisé en ce qu'il contient de 5 à 30% en poids d'un produit de revêtement selon la revendication 15.

**Revendications pour l'Etat contractant suivant : AT**

**1.** Procédé de préparation de produits de condensation qui contiennent essentiellement des propylène-urées substituées de formules générales (I) et/ou (II)

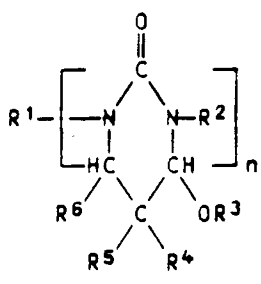

(I)

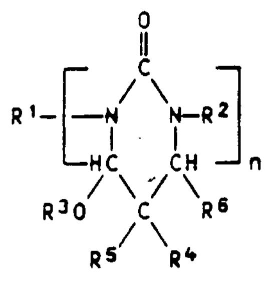

(II)

dans lesquelles $R^1$ représente un reste organique au moins bivalent alkylène, cycloalkylène, oxaalkylène ou azaalkylène, en chaîne droite ou ramifiée, contenant 4 à 60 atomes de carbone, le reste $R^1$ pouvant contenir éventuellement un ou plusieurs groupements hydroxyle et/ou groupements urée, carbamate, carbonamide ou sulfonamide substitues par des groupements hydroxyalkyle, hydroxycycloalkyle, hydroxyoxaalkyle et/ou hydroxyazaalkyle,
$R^2$, $R^3$, $R^4$, $R^5$ et $R^6$ sont identiques ou différents les uns des autres et sont mis pour des atomes d'hydrogène, des restes alkyle à 1-18 atomes de carbone, des restes cycloalkyle à 5-15 atomes de carbone, des restes aryle à 6-15 atomes de carbone, des restes aralkyle à 7-16 atomes de carbone ou des restes oxaalkyle ou azaalkyle à 2-18 atomes de carbone, $R^2$ peut en plus être mis pour un reste hydroxyalkyle, étant spécifié que dans le cas où $R^2$ est mis pour un reste hydroxyalkyle, celui-ci contient de 4 à 18 atomes de carbone, et $R^2$ n'est pas mis pour un reste aryle ou aralkyle,
et n = 2 à 20, la valence de $R^1$ correspondant à n et les restes $R^2$ à $R^6$ individuels des n motifs propylène-urée des formules (I) et/ou (II) pouvant être chaque fois identiques ou différents les uns des autres dans le cadre des définitions données ci-dessus, caractérisé en ce qu'on fait d'abord réagir

a) des di- et/ou polyamines primaires de formule générale (III)

$R^1[NH_2]_n$

avec

b) de l'urée, des urées monosubstituées ou disubstituées symétriques de formule générale (IV) $R^2NH\text{-}CO\text{-}NHR^2$,

à des températures de 100 à 200 °C, éventuellement en présence de catalyseurs acides ou basiques et éventuellement en présence

c) de composés dihydroxylés, de composés polyhydroxylés, d'alcanolamines, d'acides hydroxyalkylcarboxyliques et/ou d'acides hydroxyalkylsulfoniques ou d'esters d'acides hydroxyalkylsulfoniques,

et on fait réagir les produits ainsi obtenus avec

d) 1,4 à 2,5 moles d'un ou de plusieurs aldéhydes de formule générale (V)

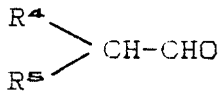 et/ou (VI) $R^6$-CHO

par mole d'urée (b), en présence de catalyseurs acides à des températures de 50 à 150 °C, éventuellement en présence d'un solvant, étant spécifié qu'on utilise, par mole d'urée (b) mise en réaction, au moins 0,7 mole d'un aldéhyde (d) qui contient au moins un atome d'hydrogène se trouvant en position $\alpha$ par rapport au groupement aldéhyde,

ou bien on fait d'abord réagir

a) des monoamines primaires de formule générale (VII) $R^2$-$NH_2$ ou des di- et/ou polyamines primaires de formule générale (III)

$R^1$$[NH_2]_n$

avec

e) des monoisocyanates de formule générale (VIII) $R^2$-NCO ou des di- et/ou polyisocyanates de formule générale (IX)

$R^1$$[NCO]_n$

étant spécifié que dans le cas où des monoamines primaires $R^2$-$NH_2$ sont utilisées comme composant (a), elles sont mises en réaction avec des di- et/ou polyisocyanates

$R^1$$[NCO]_n$

et que dans le cas où des monoisocyanates $R^2$-NCO sont utilisés comme composant (e), ils sont mis en réaction avec des di- et/ou des polyamines

$R^1$$[NH_2]_n$

et on fait réagir les di- ou polyurées ainsi obtenues avec

d) 1,4 à 2,5 moles d'un ou de plusieurs aldéhydes

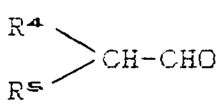 et/ou $R^6$-CHO

par mode des motifs urée formés par la réaction de (a) avec (e), en présence de catalyseurs acides à des températures de 50 à 150 °C, éventuellement en présence d'un solvant, étant spécifié qu'on utilise, par mole de motifs urée formés par reaction de (a) avec (e), au moins 0,7 mole d'au moins un aldéhyde (d) qui contient au moins un atome d'hydrogène se trouvant en position $\alpha$ par rapport au groupement aldéhyde.

**2.** Procédé selon la revendication 1, caractérisé en ce qu'on utilise, comme aldéhyde (d), de l'isobutyraldéhyde ou un mélange d'isobutyraldéhyde et de formaldéhyde.

**3.** Procédé de préparation de produits de réaction de produits de condensation qui contiennent essentiellement des propylène-urées substituées de formules générales (I) et/ou (II)

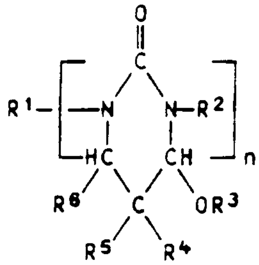

(I)

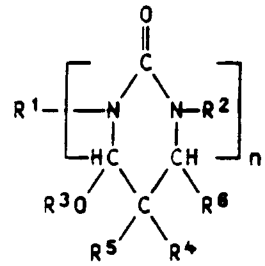

(II)

dans lesquelles $R^1$ représente un reste organique au moins bivalent alkylène, cycloalkylène, oxaalkylène ou azaalkylène, en chaîne droite ou ramifiée, contenant 4 à 60 atomes de carbone, le reste $R^1$ pouvant contenir éventuellement un au plusieurs groupements hydroxyle et/ou groupements urée, carbamate, carbonamide ou sulfonamide substitués par des groupements hydroxyalkyle, hydroxycycloalkyle, hydroxyoxaalkyle et/ou hydroxyazaalkyle,

$R^2$, $R^3$, $R^4$, $R^5$ et $R^6$ sont identiques ou différents les uns des autres et sont mis pour des atomes d'hydrogène, des restes alkyle à 1-18 atomes de carbone, des restes cycloalkyle à 5-15 atomes de carbone, des restes aryle à 6-15 atomes de carbone, des restes aralkyle à 7-16 atomes de carbone ou des restes oxaalkyle ou azaalkyle à 2-18 atomes de carbone, $R^2$ peut en plus être mis pour un reste hydroxyalkyle, étant spécifié que dans le cas où $R^2$ est mis pour un reste hydroxyalkyle, celui-ci contient de 4 à 18 atomes de carbone, et $R^2$ n'est pas mis pour un reste aryle ou aralkyle,

et n = 2 à 20, la valence de $R^1$ correspondant à n et les restes $R^2$ à $R^6$ individuels des n motifs propylène-urée des formules (I) et/ou (II) pouvant être chaque fois identiques ou différents les uns des autres dans le cadre des définitions données ci-dessus,

avec

des di- et/ou polyisocyanates,

des dérivés d'acides di- et/ou polycarboxyliques ou

des phénols p-substitués et/ou leurs dérivés o-méthylolés, étant spécifié qu'en cas d'utilisation d'o-méthylolphénols, l'un au moins des restes $R^1$ ou $R^2$ des formules (I) et/ou (II) contient un groupement hydroxyle ou le reste $R^2$ est mis pour un atome d'hydrogène,

caractérisé en ce qu'on fait d'abord réagir

a) des di- et/ou polyamines primaires de formule générale (III)

$$R^1[NH_2]_n$$

avec

b) de l'urée, des urées monosubstituées ou disubstituées symetriques de formule générale (IV) $R^2NH\text{-}CO\text{-}NHR^2$,

à des températures de 100 à 200 °C, éventuellement en présence de catalyseurs acides ou basiques et éventuellement en présence

c) de composés dihydroxylés, de composés polyhydroxylés, d'alcanolamines, d'acides hydroxyalkylcarboxyliques et/ou d'acides hydroxyalkylsulfoniques ou d'esters d'acides hydroxyalkylsulfoniques,

et on fait réagir les di- et/ou polyurées ainsi obtenues avec

d) 1,4 à 2,5 moles d'un ou de plusieurs aldéhydes de formule générale (V)

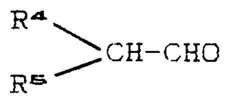 et/ou (VI) $R^6$-CHO

par mole d'urée (b), en présence de catalyseurs acides à des températures de 50 à 150 °C, éventuellement en présence d'un solvant, étant spécifié qu'on utilise, par mole d'urée (b) mise en réaction, au moins 0,7 mole d'un aldéhyde (d) qui contient au moins un atome d'hydrogène se trouvant en position $\alpha$ par rapport au groupement aldéhyde,

$R^1$ à $R^6$ et n ayant les significations indiquées dans la revendication 1,

et on fait réagir les produits ainsi obtenus avec

des di- et/ou polyisocyanates,
des dérivés d'acides di- et/ou polycarboxyliques ou
des phénols p-substitués et/ou leurs dérivés o-méthylolés, étant spécifié qu'on utilise, par mole d'urée (b) mise en réaction, de 0,25 à 1,0 mole de groupements isocyanate du di- et/ou polyisocyanate, de groupements halogénure et/ou anhydride d'acide ou de phénol p-substitué et/ou du dérivé o-méthylolé de celui-ci.
ou bien on fait d'abord réagir

a) des monoamines primaires de formule générale (VII) $R^2$-$NH_2$ ou des di- et/ou polyamines primaires de formule générale (III)

$R^1[NH_2]_n$

avec

e) des monoisocyanates de formule générale (VIII) $R^2$-NCO ou des di- et/ou polyisocyanates de formule générale (IX)

$R^1[NCO]_n$

étant spécifié que dans le cas où des monoamines primaires $R^2$-$NH_2$ sont utilisées comme composent (a), elles sont mises en réaction avec des di- et/ou polyisocyanates

$R^1[NCO]_n$

et que dans le cas où des monoisocyanates $R^2$-NCO sont utilisés comme composant (e), ils sont mis en réaction avec des di- et/ou des polyamines

$R^1[NH_2]_n$

et on fait réagir les di- ou polyurées ainsi obtenues avec

d) 1,4 à 2,5 moles d'un ou de plusieurs aldéhydes

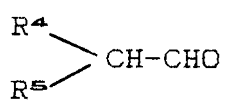 et/ou $R^6$-CHO

par mole des motifs urée formés par la réaction de (a) avec (e), en présence de catalyseurs acides à des températures de 50 à 150 ° C, éventuellement en présence d'un solvant, étant spécifié qu'on utilise, par mole de motifs urée formés par réaction de (a) avec (e), au moins 0,7 mole d'au moins un aldéhyde (d) qui contient au moins un atome d'hydrogène se trouvant en position $\alpha$ par rapport au groupement aldéhyde,

$R^1$ à $R^6$ et n ayant les significations indiquées dans la revendication 1,
et an fait réagir les produits ainsi obtenus avec
des di- et/ou polyisocyanates,
des dérivés d'acides di- et/au polycarboxyliques ou
des phénols p-substitués et/ou leurs dérivés o-méthylolés, étant spécifié qu'on utilise, par mole d'urée (b) mise en réaction, de 0,25 à 1,0 mole de groupements isocyanate du di- et/ou polyisocyanate, de groupements halogénure et/ou anhydride d'acide ou de phénol p-substitué et/ou du dérivé o-méthylolé de celui-ci.

**4.** Procédé selon la revendication 3, caractérisé en ce qu'on utilise, comme aldéhyde (d), de l'isobutyraldéhyde ou un mélange d'isobutyraldéhyde et de formaldéhyde.

**5.** Procédé de préparation d'un produit de revêtement thermodurcissable, caractérisé en ce qu'on utilise, en tant que liant, un mélange

(A) d'au moins un produit de polyaddition, de polycondensation ou de polymérisation ayant un poids moléculaire moyen $\overline{M}_n$ de 500 à 10 000, qui renferme en moyenne, par molécule, au moins deux groupements hydroxyle et éventuellement et en plus des groupements amino primaires et/ou

secondaires et/ou tertiaires, et
(B) d'un produit de condensation préparé par un procédé selon la revendication 1.

**6.** Procédé de préparation d'un produit de revêtement thermodurcissable, caractérise en ce qu'on utilise, en tant que liant, un mélange
(A) d'au moins un produit de polyaddition, de polycondensation ou de polymérisation ayant un poids moléculaire moyen $\overline{M}_n$ de 500 à 10 000, qui renferme en moyenne, par molécule, au moins deux groupements hydroxyle et éventuellement et en plus des groupements amino primaires et/ou secondaires et/ou tertiaires, et
(B) d'un produit de réaction préparé par un procédé selon la revendication 3.

**7.** Procédé selon la revendication 5 ou 6, caractérisé en ce qu'un utilise, comme composant (A), une résine de polyester.

**8.** Procédé selon la revendication 5 ou 6, caractérisé en ce qu'on utilise, comme composant (A), un produit de réaction d'une résine époxy avec au moins une amine, un alcool ou un mercaptan.

**9.** Procédé selon la revendication 5 ou 6, caractérisé en ce qu'on utilise, comme composant (A), une résine polyacrylique contenant des groupements hydroxyle.

**10.** Procédé de préparation de vernis à cuire aqueux et de vernis de trempage électrophorétique à dépôt cathodique, caractérisé en ce que les produits de revêtement thermodurcissables préparés par un procédé selon l'une quelconque des revendications 5 à 9 sont rendus diluables à l'eau par neutralisation, au moins partielle, par un acide.